# EUROPEAN PATENT APPLICATION

(11) **EP 4 817 589 A1**
(43) Date of publication of application: **07.10.2026**
(21) Application number: 24897669.8
(22) Date of filing: 28.11.2024
(51) Int. Cl.: A61K 6/818, A61C 5/70, A61C 13/083, A61K 6/30, A61K 6/824, C04B 35/486

(54) **ZIRCONIA COMPOSITE SINTERED BODY AND DENTAL PRESERVATION REPAIR KIT INCLUDING SAME**

(30) Priority: 28.11.2023 JP 2023201151
(71) Applicant: Kuraray Noritake Dental Inc., Kurashiki-shi, Okayama 710-0801 (JP)
(72) Inventor: NIWA, Takahiro, Miyoshi-shi, Aichi 470-0293 (JP); NAKANO, Kirihiro, Miyoshi-shi, Aichi 470-0293 (JP); SAKAMOTO, Hiroyuki, Miyoshi-shi, Aichi 470-0293 (JP); KASHIKI, Nobusuke, Miyoshi-shi, Aichi 470-0293 (JP)
(74) Representative: D Young & Co LLP
(86) International application number: PCT/JP2024/042237
(87) International publication number: WO 2025/115985

(57) **Abstract**

The present invention provides a machinable zirconia composite sintered body capable of exhibiting excellent adhesive properties without sandblasting after machining, as well as a dental kit for conservative restoration including same. The present invention relates to a dental zirconia composite sintered body comprising monoclinic zirconia and having a monoclinic abundance fₘ of more than 10% and 60% or less as represented by the following formula (1), ${f}_{m} = {l}_{28} / \left({l}_{28}+{l}_{30}\right) \times 100$ (1), wherein fₘ represents the monoclinic abundance (%), I₂₈ represents the integrated intensity of the peak near 2θ = 28°, where the main peak of the monoclinic zirconia appears in XRD measurement, and I₃₀ represents the integrated intensity of the peak near 2θ = 30°, where the main peak of tetragonal or cubic zirconia appears in XRD measurement.

## Description

### TECHNICAL FIELD

The present invention relates to a zirconia composite sintered body, and a dental kit for conservative restoration including same.

More specifically, the present invention relates to a machinable zirconia composite sintered body having excellent adhesive properties, and a dental kit for conservative restoration including same.

### BACKGROUND ART

Ceramics made from metal oxides are used in a wide range of industrial applications. Notably, zirconia sintered bodies have found use in dental materials, such as dental prostheses, due to their high strength and aesthetic qualities.

Because of superior strength, zirconia sintered bodies hardly involve issues such as damage when used in dental materials such as prostheses. Zirconia sintered bodies also have high translucency and resistance to staining in the oral cavity, leading to their superior aesthetic qualities.

Concerning such dental material applications, for example, a dental prosthesis can be produced by acquiring a patient's intraoral data and using this data to machine a dental zirconia material with a CAD/CAM system.

Machining of a dental zirconia material with a CAD/CAM system is typically performed in a dental laboratory or dental clinic equipped with a CAD/CAM system, where a zirconia pre-sintered body is machined into a crown shape and sintered for use as a zirconia sintered body.

Zirconia sintered bodies with various improvements have been proposed, with consideration given to production efficiency and other factors (for example, Patent Literatures 1 and 2).

### CITATION LIST

### Patent Literature

Patent Literature 1: JP2021-88501 A
Patent Literature 2: JP2023-37381 A

### SUMMARY OF INVENTION

### Technical Problem

Zirconia sintered bodies formed through processing into a crown shape and subsequent sintering have few surface irregularities. For this reason, clinically adequate adhesion has traditionally been achieved by sandblasting the surface of the zirconia sintered body to be bonded to a supporting tooth (hereinafter, also referred to as "bonding surface"), and thereby increasing its surface area (for example, Patent Literature 1).

Consequently, the zirconia sintered body according to Patent Literature 1 fails to provide sufficient bond strength in the absence of sandblasting.

However, sandblasting involves inconsistency in the level of treatment depending on the practitioner, and, in some instances, this has resulted in insufficient adhesion.

The present inventors have also found that the zirconia sintered body according to Patent Literature 2 cannot achieve sufficient bond strength without sandblasting.

It is an object of the present invention to provide a machinable zirconia composite sintered body capable of exhibiting excellent adhesive properties without sandblasting after machining, as well as a dental kit for conservative restoration including same.

### Solution to Problem

The present inventors conducted intensive studies to find a solution to the foregoing problem, and found that this issue can be solved by setting the monoclinic fraction at the machined surface of a zirconia composite sintered body within a specific range. Building upon this finding, the present inventors conducted additional examinations, ultimately resulting in the completion of the present invention.

Specifically, the present invention includes the following.
[1] A dental zirconia composite sintered body comprising monoclinic zirconia and having a monoclinic abundance fₘ of more than 10% and 60% or less as represented by the following formula (1),${f}_{m} = {I}_{28} / \left({I}_{28}+{I}_{30}\right) \times 100$ (1), wherein fₘ represents the monoclinic abundance (%), I₂₈ represents the integrated intensity of the peak near 2θ = 28°, where the main peak of the monoclinic zirconia appears in XRD measurement, and I₃₀ represents the integrated intensity of the peak near 2θ = 30°, where the main peak of tetragonal or cubic zirconia appears in XRD measurement.
[2] The dental zirconia composite sintered body according to [1], wherein the monoclinic zirconia is present at the surface.
[3] The dental zirconia composite sintered body according to [1] or [2], which has a surface roughness Ra of 3.0 to 8.0 µm.
[4] The dental zirconia composite sintered body according to any one of [1] to [3], which has an average crystal grain size of 0.5 to 10 µm.
[5] The dental zirconia composite sintered body according to any one of [1] to [4], which further comprises a stabilizer capable of preventing a phase transformation of zirconia.
[6] The dental zirconia composite sintered body according to [5], wherein the content of the stabilizer is 1 to 12 mol% in total 100 mol% of zirconia and the stabilizer.
[7] The dental zirconia composite sintered body according to [5] or [6], wherein the stabilizer is yttria.
[8] The dental zirconia composite sintered body according to any one of [5] to [7], which further comprises Nb₂O₅ and/or Ta₂O₅.
[9] The dental zirconia composite sintered body according to any one of [5] to [8], wherein the content of the Nb₂O₅ and/or Ta₂O₅ is 1 to 9 mol% in total 100 mol% of zirconia, the stabilizer, Nb₂O₅, and Ta₂O₅.
[10] The dental zirconia composite sintered body according to [8] or [9], which further comprises an element or ion derived from a capping agent.
[11] The dental zirconia composite sintered body according to [10], wherein the content of the element or ion derived from a capping agent is more than 0 mol% and 5 mol% or less relative to total 100 mol% of zirconia, the stabilizer, Nb₂O₅, and Ta₂O₅.
[12] The dental zirconia composite sintered body according to [10] or [11], wherein the element or ion derived from a capping agent belongs to periods 2 to 7 of the periodic table, and is an element or an ion thereof having a first ionization energy lower than that of a group 18 element of the same period, and/or an element or an ion thereof having a high electron affinity.
[13] The dental zirconia composite sintered body according to [10], wherein the element or ion derived from a capping agent comprises at least one element or an ion thereof selected from the group consisting of Cu, Ag, Li, Na, K, Rb, Cs, Fr, At, I, Br, Cl, and F.
[14] The dental zirconia composite sintered body according to [11], wherein the element or ion derived from a capping agent comprises at least one element selected from the group consisting of Li, Na, and K.
[15] The dental zirconia composite sintered body according to any one of [8] to [14], which further comprises TiO₂ and/or Al₂O₃.
[16] A dental kit for conservative restoration, comprising the dental zirconia composite sintered body of any one of [1] to [15] in combination with a dental cement.
[17] The dental kit for conservative restoration according to [16], wherein the dental cement comprises an acidic component.
[18] The dental kit for conservative restoration according to [17], wherein the acidic component comprises a monomer having an acidic group.
[19] The dental kit for conservative restoration according to [18], wherein the monomer having an acidic group comprises at least one selected from the group consisting of a phosphoric acid group, a pyrophosphoric acid group, a thiophosphoric acid group, a phosphonic acid group, a carboxylic acid group, and a sulfonic acid group.
[20] A method for bonding the dental zirconia composite sintered body of any one of [1] to [15], comprising bonding using a dental cement, without sandblasting.

### Advantageous Effects of Invention

According to the present invention, a machinable zirconia composite sintered body can be provided that is capable of exhibiting excellent adhesive properties without sandblasting after machining, as well as a dental kit for conservative restoration including same.

Because a machinable zirconia composite sintered body of the present invention can exhibit excellent adhesive properties without sandblasting after machining, it can achieve even higher adhesive properties (particularly, bond durability) when subjected to sandblasting.

### BRIEF DESCRIPTION OF DRAWINGS

[FIG. 1] An XRD measurement result showing that the dental zirconia composite sintered body according to Example 1 includes monoclinic zirconia after machining.
[FIG. 2] A partially enlarged view of FIG. 1.

### DESCRIPTION OF EMBODIMENTS

A dental zirconia composite sintered body of the present invention (hereinafter, also referred to simply as "zirconia composite sintered body") comprises monoclinic zirconia and has a monoclinic abundance fₘ (hereinafter, also referred to as "monoclinic fraction") of more than 10% and 60% or less as represented by the following formula (1),${f}_{m} = {I}_{28} / \left({I}_{28}+{I}_{30}\right) \times 100$ (1), wherein fₘ represents the monoclinic abundance (%), I₂₈ represents the integrated intensity of the peak near 2θ = 28°, where the main peak of the monoclinic zirconia appears in XRD measurement, and I₃₀ represents the integrated intensity of the peak near 2θ = 30°, where the main peak of tetragonal or cubic zirconia appears in XRD measurement.

As used herein, "molded body" refers to a state in which neither a semi-sintered state (pre-sintered state) nor a sintered state has been reached. That is, "molded body" distinguishes itself from pre-sintered body and sintered body in that it remains unfired after being molded.

As used herein, "zirconia composite pre-sintered body" refers to a semi-sintered state in which a raw material powder such as zirconia has formed necks but has not fully sintered.

As used herein, "zirconia composite sintered body" refers to a sintered state in which a raw material powder such as zirconia has fully sintered. In a zirconia composite sintered body, sintering has caused a raw material powder such as zirconia to consolidate or progressively form a solid solution, increasing the relative density and promoting densification, resulting in a fully sintered state with a relative density of 95% or more.

As used herein, "zirconia" refers to zirconium(IV) oxide (ZrO₂), with a trace amount (0.5 mass% or more and 3 mass% or less) of HfO₂ relative to the amount of ZrO₂. Because HfO₂ is difficult to separate, terms such as "zirconia" and "zirconia powder" are intended to include both ZrO₂ and HfO₂. Likewise, a powder with a stabilizer present in zirconia as a solid solution is also encompassed by "zirconia powder."

As used herein, "in the atmosphere" means under standard atmospheric pressure (1 atm).

As used herein, "zirconia enhancer" refers to a component that serves to enhance the mechanical strength of the zirconia composite sintered body.

As used herein, "total 100 mol% of zirconia, the stabilizer, Nb₂O₅, and Ta₂O₅" can be read as referring to "total 100 mol% of zirconia and the stabilizer" in a zirconia composite sintered body that comprises neither Nb₂O₅ nor Ta₂O₅.

As used herein, the term "(meth)acryl" is intended to be inclusive of both methacryl and acryl, and the same applies to similar expressions such as "(meth)acrylate", "(meth)acrylic acid ester", "(meth)acrylamide", and "(meth)acryloyloxy."

In this specification, the content of each component in the zirconia composite sintered body can be calculated from the amounts of raw materials charged.

In this specification, machining encompasses both cutting and grinding. Machining may be a wet or dry process, without specific restrictions.

As used herein, "surface roughness Ra" refers to the arithmetic mean roughness according to JIS B 0601:2013.

As used herein, "mean spacing of irregularities RSm" refers to the mean spacing of irregularities according to JIS B 0601:2001.

In this specification, the upper limits and lower limits of numeric ranges (including, for example, temperature ranges, the content of each component, the abundance of crystal systems, components, values calculated from components, and various physical properties) can be appropriately combined.

The following speculation has been made as to why a zirconia composite sintered body of the present invention exhibits excellent adhesive properties (particularly, bond durability) without sandblasting after machining.

In a zirconia composite sintered body of the present invention, external forces applied during machining induce the formation of fine cracks, which cause a phase transformation to occur in the crystal system from tetragonal to monoclinic, at least at the surface. The amount of fine cracking is proportional to the monoclinic fraction, meaning that a higher monoclinic fraction indicates a greater number of fine cracks and higher bond durability.

A zirconia composite sintered body of the present invention can achieve superior adhesive properties (particularly, bond durability) with a monoclinic abundance fₘ of more than 10% and 60% or less.

In view of increasing fine cracking and providing even superior adhesive properties (particularly, bond durability), the monoclinic abundance fₘ is preferably more than 15%, more preferably 17% or more, even more preferably 18% or more, particularly preferably 20% or more, most preferably 21% or more.

In view of confining fine cracking within an appropriate range and providing even superior adhesive properties (particularly, bond durability), the monoclinic abundance fₘ is preferably 58% or less, more preferably 55% or less, even more preferably 50% or less, particularly preferably 45% or less, most preferably 40% or less.

The method of measurement of monoclinic abundance fₘ is as described in the EXAMPLES below.

Preferably, the monoclinic zirconia is present at the surface. The zirconia composite sintered body can achieve even superior adhesive properties (particularly, bond durability) when the monoclinic zirconia is present at the surface with the abundance fₘ specified above.

The surface roughness Ra represents the arithmetic mean value of surface irregularities in the height direction.

In view of enhancing the anchoring effect on adhesives and providing improved bond strength and even superior adhesive properties (particularly, bond durability), a zirconia composite sintered body of the present invention has a surface roughness Ra of preferably 3.0 µm or more, more preferably 3.5 µm or more, even more preferably 4.0 µm or more, particularly preferably 4.5 µm or more at the surface.

In view of reducing surface embrittlement and ensuring strength as a dental prosthesis while providing even superior adhesive properties (particularly, bond durability), the surface roughness Ra is preferably 8.0 µm or less, more preferably 7.5 µm or less, even more preferably 7.0 µm or less, particularly preferably 6.5 µm or less.

The method of measurement of surface roughness Ra is as described in the EXAMPLES below.

An increase in surface roughness Ra indicates deeper surface irregularities, which permits the adhesive to infiltrate further into the surface, strengthening the anchoring effect and increasing adhesive strength.

The mean spacing of irregularities RSm represents the measured distance between irregularities as observed from the side in a cross-sectional view of the rough surface.

In view of enhancing the anchoring effect on adhesives and providing improved bond strength and even superior adhesive properties (particularly, bond durability), a zirconia composite sintered body of the present invention has a mean spacing of irregularities RSm of preferably 50 µm or more, more preferably 100 µm or more, even more preferably 150 µm or more, particularly preferably 200 µm or more, most preferably 250 µm or more at the surface.

In view of reducing surface embrittlement and ensuring strength as a dental prosthesis while providing even superior adhesive properties (particularly, bond durability), the mean spacing of irregularities RSm is preferably 700 µm or less, more preferably 600 µm or less, even more preferably 500 µm or less, particularly preferably 450 µm or less, most preferably 400 µm or less.

The method of measurement of mean spacing of irregularities RSm is as described in the EXAMPLES below.

A decrease in mean spacing of irregularities RSm indicates smaller distances between irregularities, meaning that the surface has fine asperities with a large specific surface area, leading to increased adhesive strength.

In view of providing spaces permitting infiltration of an adhesive and achieving even superior adhesive properties (particularly, bond durability), the degree of liquid penetration at the surface of a zirconia composite sintered body of the present invention is preferably 0.5 mm or more, more preferably 1.5 mm or more, even more preferably 2.0 mm or more, particularly preferably 2.5 mm or more, most preferably 3.0 mm or more.

A higher degree of penetration leads to the formation of numerous fine cracks at the surface, permitting more liquid to infiltrate the fine spaces. Presumably, this allows a liquid adhesive to penetrate deeper into the surface layer, enhancing the adhesive anchoring effect and thereby providing improved bond strength and even superior adhesive properties (particularly, bond durability).

In view of reducing surface embrittlement and ensuring strength as a dental prosthesis while providing even superior adhesive properties (particularly, bond durability), the degree of liquid penetration at the surface is preferably 7 mm or less, more preferably 6.5 mm or less, even more preferably 6 mm or less, particularly preferably 5.5 mm or less, most preferably 5 mm or less.

The method of measurement of degree of penetration is as described in the EXAMPLES below.

With the degree of penetration falling in these ranges, it is possible to achieve excellent adhesive properties while ensuring strength as a dental prosthesis, together with the monoclinic abundance fₘ.

Throughout this specification, descriptions concerning a zirconia composite sintered body of the present invention are applicable to all embodiments, except where explicitly indicated otherwise.

In a zirconia composite sintered body of the present invention, the zirconia content is preferably 78 to 97.5 mol% in total 100 mol% of zirconia, the stabilizer, Nb₂O₅, and Ta₂O₅. In view of even superior translucency and strength, the zirconia content is more preferably 79 mol% or more and 96 mol% or less, even more preferably 80 mol% or more and 94 mol% or less, particularly preferably 81 mol% or more and 93 mol% or less.

In a zirconia composite sintered body of the present invention, examples of the stabilizer capable of preventing a phase transformation of zirconia (hereinafter, also referred to as "stabilizer") include oxides such as calcium oxide (CaO), magnesium oxide (MgO), yttrium oxide (Y₂O₃), cerium oxide (CeO₂), scandium oxide (Sc₂O₃), lanthanum oxide (La₂O₃), erbium oxide (Er₂O₃), praseodymium oxide (Pr₂O₃, Pr₆O₁₁), samarium oxide (Sm₂O₃), europium oxide (Eu₂O₃), thulium oxide (Tm₂O₃), gallium oxide (Ga₂O₃), indium oxide (In₂O₃), and ytterbium oxide (Yb₂O₃). In view of achieving superior machinability and strength in the sintered state and providing a dental prosthesis that exhibits notably superior aesthetics with an excellent appearance more closely resembling natural teeth after machining when combined with components containing at least one of Nb₂O₅ or Ta₂O₅, more preferred are Y₂O₃ (yttria) and/or CeO₂, even more preferably Y₂O₃. The stabilizer may be used alone, or two or more thereof may be used in combination.

In a zirconia composite sintered body of the present invention, the stabilizer content is preferably 1 to 12 mol% in total 100 mol% of zirconia, the stabilizer, Nb₂O₅, and Ta₂O₅. In view of ease of achieving sufficient machinability, the stabilizer content is more preferably 2 mol% or more and 10 mol% or less. In view of even superior translucency and strength, the stabilizer content is even more preferably 3 mol% or more and 8.0 mol% or less, yet more preferably 3.5 mol% or more and 7.5 mol% or less, particularly preferably 3.8 mol% or more and 7.0 mol% or less, most preferably 4.0 mol% or more and 6.5 mol% or less.

In embodiments where the zirconia composite sintered body comprises neither Nb₂O₅ nor Ta₂O₅, the stabilizer content refers to the content in total 100 mol% of zirconia and the stabilizer.

Preferred embodiments of a zirconia composite sintered body of the present invention are described below.

In view of providing superior machinability in the sintered state, it is preferable that a zirconia composite sintered body of the present invention comprise Nb₂O₅ and/or Ta₂O₅, in addition to zirconia and a stabilizer capable of preventing a phase transformation of zirconia.

A certain preferred embodiment is, for example, a zirconia composite sintered body comprising zirconia, a stabilizer capable of preventing a phase transformation of zirconia, and at least one of Nb₂O₅ or Ta₂O₅.

The reason why a zirconia composite sintered body of the present invention exhibits superior machinability in the sintered state while maintaining strength is unclear. However, by additionally incorporating at least one of Nb₂O₅ or Ta₂O₅ along with conventionally stabilized zirconia, it is considered possible to improve fracture toughness (IF method) while minimizing hardness through coarsening of the microstructure.

It is also presumed that the additional incorporation of at least one of Nb₂O₅ or Ta₂O₅ promotes the formation of fine cracks upon every application of external forces during machining through reactions with the stabilizer, facilitating a phase transformation of the crystal system from tetragonal to monoclinic at least at the surface, thereby making it easier to adjust the monoclinic abundance fₘ to the desired range.

In a zirconia composite sintered body of the present invention, the content of Nb₂O₅ or Ta₂O₅ is preferably 1 to 9 mol%, more preferably 1.5 mol% or more and 8.5 mol% or less in total 100 mol% of zirconia, the stabilizer, Nb₂O₅, and Ta₂O₅. In view of providing even superior machinability in the sintered state, the content of Nb₂O₅ or Ta₂O₅ is even more preferably 2.5 mol% or more and 8.0 mol% or less, yet more preferably 2.7 mol% or more and 7.0 mol% or less, particularly preferably 2.8 mol% or more and 6.0 mol% or less, most preferably 3.0 mol% or more and 5.5 mol% or less.

With the content of Nb₂O₅ or Ta₂O₅ falling in these ranges, it is possible to achieve sufficient machinability in the sintered state while reducing the occurrence of defects such as chipping.

In view of superior machinability in the sintered state, it is preferable that a zirconia composite sintered body of the present invention comprise an element or ion derived from a capping agent, in addition to zirconia and a stabilizer capable of preventing a phase transformation of zirconia.

Another certain preferred embodiment is, for example, a zirconia composite sintered body that comprises zirconia, a stabilizer capable of preventing a phase transformation of zirconia, and a capping element or ion.

In such an embodiment, the zirconia composite sintered body may comprise at least one of Nb₂O₅ or Ta₂O₅, or neither Nb₂O₅ nor Ta₂O₅.

In view of particularly superior machinability in the sintered state, the following description will be given through the case of a zirconia composite sintered body that comprises zirconia, a stabilizer capable of preventing a phase transformation of zirconia, at least one of Nb₂O₅ or Ta₂O₅, and a capping element or ion.

In view of particularly superior machinability, it is preferable that a zirconia composite sintered body of the present invention comprise zirconia, a stabilizer capable of preventing a phase transformation of zirconia, at least one of Nb₂O₅ or Ta₂O₅, and an element or ion derived from a capping agent.

In this specification, elements or ions derived from a capping agent (hereinafter, also referred to as "capping elements or ions") refer to elements or ions that weaken the strength of the crystal interface (hereinafter, also referred to as "grain boundary") by capping the terminals of the bonding sites of zirconia-based composite oxides within a zirconia composite sintered body constituted of zirconia-based composite oxides (hereinafter, the strength of grain boundaries is also referred to as "grain boundary strength").

The capping agent can at least partially cap the crystal grain boundaries.

The term "capping" refers to elements or ions (capping elements or ions) of interest existing at the crystal grain boundaries by binding to the bonding sites of zirconia-based composite oxides instead of the metal elements.

It is believed that the presence of capping elements or ions in the form of +1 cations or -1 anions at the grain boundaries causes electrostatic repulsion between these capping cations or anions, thereby reducing the grain boundary strength.

It is also presumed that the additional incorporation of capping elements or ions promotes the formation of fine cracks upon every application of external forces during machining.

The content of zirconia, stabilizer, Nb₂O₅, and Ta₂O₅ in the zirconia composite sintered body can be individually measured using a technique, for example, such as inductively coupled plasma (ICP) emission spectral analysis or X-ray fluorescence analysis.

The content (mol%) of elements or ions derived from a capping agent refers to the proportion external to total 100 mol% of zirconia, the stabilizer, Nb₂O₅, and Ta₂O₅. Accordingly, the content of the elements or ions derived from a capping agent in the zirconia composite sintered body can be calculated by converting the quantity (mass) of the raw material added into mol%.

The zirconia composite sintered body comprising zirconia, a stabilizer capable of preventing a phase transformation of zirconia, at least one of Nb₂O₅ or Ta₂O₅, and an element or ion derived from a capping agent exhibits excellent machinability in the sintered state while having strength and translucency suited for dental use. It remains unclear why such a zirconia composite sintered body allows for machining in the sintered state. However, the following speculation has been made.

When present at the crystal grain boundaries in the zirconia composite sintered body comprising zirconia and the stabilizer (particularly preferably, a zirconia composite sintered body comprising Nb₂O₅ and/or Ta₂O₅), the capping elements or ions appear to reduce the grain boundary strength in the form of +1 cations or -1 anions, facilitating particles to separate, thereby improving grindability and machinability.

In the form of +1 cations or -1 anions, the capping elements or ions bind to the bonding sites of zirconia-based composite oxides at the grain boundaries of the zirconia composite sintered body. This binding leads to electrostatic repulsion between the cations or anions, reducing the grain boundary strength while maintaining the strength and translucency properties of the particles constituting the zirconia composite sintered body, resulting in improved machinability.

As a possible example of such bonding, a +1 cation can form a bond with the other bonding site of an oxygen atom that is already bonded to the metal element (for example, Zr, Hf, Y, Nb, or Ta) contained in the zirconia-based composite oxide, instead of these metal elements forming such bonds.

Another possible example is where a -1 anion binds to the OH₂⁺ attached to the metal element (for example, Zr, Hf, Y, Nb, or Ta) contained in the zirconia-based composite oxide.

In yet another possible example, a -1 anion can bind to the cation derived from the metal element (for example, Zr, Hf, Y, Nb, or Ta) contained within the zirconia-based composite oxide and attached to other metal elements.

In the zirconia composite sintered body, Nb₂O₅ and/or Ta₂O₅ serve to coarsen the microstructure and reduce hardness, and, by acting together with the capping elements or ions, further improve machinability in the sintered state. Through this combined action, Nb₂O₅ and/or Ta₂O₅ and the capping elements or ions can provide even superior free-machinability while ensuring the strength needed as artificial teeth, reducing machining time and increasing the number of dental prostheses that can be produced in continuous processing with a single processing tool while reducing wear on processing tools.

In a zirconia composite sintered body of the present invention, the capping elements or ions further improve machinability in the sintered state, and serve as a free-cutting aid in the manner described above, without greatly compromising strength and translucency.

The content of the capping elements or ions contained in a zirconia composite sintered body of the present invention is preferably more than 0 mol% and 5 mol% or less. In view of providing even superior machinability in the sintered state and increasing the number of dental prostheses that can be produced in continuous processing with a single processing tool, the content of capping elements or ions is more preferably 0.05 mol% or more and 3 mol% or less, even more preferably 0.06 mol% or more and 2.5 mol% or less, particularly preferably 0.07 mol% or more and 1.0 mol% or less, most preferably 0.08 mol% or more and 0.34 mol% or less.

When the capping elements or ions contained in a zirconia composite sintered body of the present invention are group 17 elements or ions, the content of capping elements or ions is more preferably 0.2 mol% or more and 5 mol% or less, even more preferably 0.3 mol% or more and 4 mol% or less, particularly preferably 0.4 mol% or more and 3.5 mol% or less, most preferably 0.5 mol% or more and 3.0 mol% or less in view of providing even superior machinability in the sintered state and increasing the number of dental prostheses that can be produced in continuous processing with a single processing tool.

In view of ensuring appropriate interactions between charged sites and adsorption sites at the grain boundaries, it is important that the capping elements or ions exist at the grain boundaries in the form of +1 cations or -1 anions, as noted above.

For capping elements or ions, it is preferable to use elements or ions thereof that belong to periods 2 to 7 of the periodic table, and are elements or ions thereof having a lower first ionization energy than the elements in group 18 of the same periods, or elements or ions thereof with high electron affinity. Also preferred are nitrate ions, hypochlorite ions, chlorite ions, chlorate ions, perchlorate ions, bromate ions, permanganate ions, metaborate ions, and cyanide ions.

A certain preferred embodiment is, for example, a zirconia composite sintered body in which the elements or ions thereof derived from a capping agent belong to periods 2 to 7 of the periodic table, and are elements or ions thereof having a lower first ionization energy than the elements in group 18 of the same periods, and/or elements or ions thereof with high electron affinity.

In view of facilitating the formation of +1 cations and providing even superior machinability, the elements belonging to periods 2 to 7 of the periodic table and having a lower first ionization energy than the elements in group 18 of the same periods are preferably Cu, Ag, Li, Na, K, Rb, Cs, and Fr, for example.

In view of facilitating the formation of -1 anions and providing even superior machinability, the elements with high electron affinity are preferably group 17 elements. Preferred as group 17 elements are At, I, Br, Cl, and F.

The first ionization energy refers to the energy needed to remove one electron from a neutral atom to ionize it. The first ionization energy can be referenced from Shriver & Atkins Inorganic Chemistry (Volume 1), 4th Edition, Part 1: Foundations, 1. Atomic Structure. The first ionization energy, given in eV in Appendix 2 of this book, can be converted into KJ/mol using the conversion rate of 1eV = 96.485 KJ/mol. The first ionization energy can also be determined using photoelectron yield spectroscopy (PYS).

Electron affinity (EA) is the amount of energy released when an electron is incorporated in a neutral atom. Electron affinity can be determined by the difference between the ionization potential and the energy gap. The ionization potential is defined as the energy difference between the highest occupied molecular orbital of a compound and the vacuum level, and it is measured using ultraviolet photoelectron spectroscopy.

For the first ionization energy and electron affinity, data available in the NIST Chemistry WebBook (https://webbook.nist.gov/chemistry/) may be utilized (by selecting either Ionization Energy or Electron Affinity from Ion energetics properties).

The methods described above can be appropriately employed for the measurement of first ionization energy and electron affinity because the measurement simply involves comparing the likelihood to form +1 cations or -1 anions among different elements.

Specific examples of the capping elements include Cu, Ag, Li, Na, K, Rb, Cs, Fr, At, I, Br, Cl, and F. In view of the ability to more greatly improve machinability in the sintered state, preferred are Cu, Ag, Li, Na, K, Rb, Cs, Fr, I, Br, Cl, and F.

A certain preferred embodiment is, for example, a zirconia composite sintered body in which the elements derived from a capping agent comprise at least one element selected from the group consisting of Cu, Ag, Li, Na, K, Rb, Cs, Fr, At, I, Br, Cl, and F, and ions of these elements are at least one +1 cation or -1 anion selected from the group consisting of Cu, Ag, Li, Na, K, Rb, Cs, Fr, I, Br, Cl, and F.

Another preferred embodiment is, for example, a zirconia composite sintered body in which the elements or ions derived from a capping agent comprise at least one element or ion thereof selected from the group consisting of Ag, Li, Na, K, Rb, Cs, Fr, At, I, Br, Cl, and F.

The capping elements or ions may be used alone, or two or more thereof may be used in combination.

As described above, the capping elements or ions act together with Nb₂O₅ and/or Ta₂O₅, and these do not compromise the effectiveness of the stabilizer. Accordingly, the present invention can exhibit its effects without particularly restricting the choice of stabilizer.

In addition to serving to coarsen the microstructure and reduce hardness, and acting together with the capping elements or ions to provide excellent free-machinability as noted above, Nb₂O₅ and Ta₂O₅ also interact with other components (for example, TiO₂, Al₂O₃) incorporated in the zirconia composite sintered body. This interaction, combined with the application of HIP, helps maximize the sintering density, providing natural tooth aesthetics.

The content of zirconia, stabilizer, Nb₂O₅, and Ta₂O₅ represents the proportion of each component in total 100 mol% of zirconia, stabilizer, Nb₂O₅, and Ta₂O₅, and the sum of zirconia, stabilizer, Nb₂O₅, and Ta₂O₅ does not exceed 100 mol%. For example, when the raw material composition contains Nb₂O₅ but does not contain Ta₂O₅, the content of zirconia, stabilizer, and Nb₂O₅ refers to the proportion of each component relative to total 100 mol% of zirconia, stabilizer, and Nb₂O₅.

In view of machinability, the ratio A/B is preferably 0.9 to 3, more preferably 0.95 to 2, where A is the content of stabilizer in mol%, and B is the combined content of Nb₂O₅ and Ta₂O₅ in mol%. Even more preferably, the ratio A/B is 1.0 to 1.6 in view of enhancing the effectiveness of the combined action between the capping elements or ions and Nb₂O₅ and/or Ta₂O₅, improving free-machinability, and increasing the number of dental prostheses that can be produced in continuous processing with a single processing tool while reducing wear on processing tools.

A certain preferred embodiment of the present invention is, for example, a zirconia composite sintered body in which the zirconia content is 78 to 97.5 mol%, the stabilizer content is 1 to 12 mol%, and the combined content of Nb₂O₅ and Ta₂O₅ is 1 to 9 mol% relative to the total moles of zirconia, the stabilizer, Nb₂O₅, and Ta₂O₅ in total 100 mol% of zirconia, the stabilizer, Nb₂O₅, and Ta₂O₅,
wherein the zirconia composite sintered body further comprises a capping element or ion,
the stabilizer comprises Y₂O₃ and/or CeO₂,
the content of the capping element or ion is more than 0 mol% and 5 mol% or less relative to total 100 mol% of zirconia, the stabilizer, Nb₂O₅, and Ta₂O₅, and
the zirconia composite sintered body has a ratio A/B of 0.9 to 3, where A is the content of the stabilizer in mol%, and B is the combined content of Nb₂O₅ and Ta₂O₅ in mol%.

In view of even superior strength, the zirconia composite sintered body may comprise a zirconia enhancer.

Examples of the zirconia enhancer include TiO₂ and Al₂O₃. The zirconia enhancer may be used alone, or two or more thereof may be used in combination.

A certain embodiment of the present invention is, for example, a zirconia composite sintered body that comprises zirconia, the stabilizer, and at least one of Nb₂O₅ or Ta₂O₅,
wherein the zirconia composite sintered body further comprises a zirconia enhancer in addition to zirconia, the stabilizer, Nb₂O₅ and/or Ta₂O₅, and the capping element or ion, relative to the total moles of zirconia, the stabilizer, Nb₂O₅, and Ta₂O₅.

In the zirconia composite sintered body comprising zirconia, the stabilizer, and Nb₂O₅ and/or Ta₂O₅, the zirconia enhancer can improve the strength of the sintered body through combined action with capping elements or ions.

In a zirconia composite sintered body comprising a zirconia enhancer, it is also possible to appropriately modify the zirconia content, the type and content of stabilizer, the combined content of Nb₂O₅ and Ta₂O₅, the type and content of capping elements or ions, and the ratio A/B, as noted above.

In a zirconia composite sintered body comprising a zirconia enhancer, the content of zirconia enhancer is preferably more than 0 mass% and 5.0 mass% or less relative to total 100 mass% of zirconia, the stabilizer, Nb₂O₅, and Ta₂O₅. In view of achieving even greater strength together with capping elements or ions when combined with capping elements or ions, the content of zirconia enhancer is more preferably 0.01 mass% or more and 4.5 mass% or less, even more preferably 0.5 mass% or more and 4.0 mass% or less.

The content (mass%) of zirconia enhancer refers to the proportion external to total 100 mass% of zirconia, the stabilizer, Nb₂O₅, and Ta₂O₅. Accordingly, the content of the zirconia enhancer in the zirconia composite sintered body can be calculated from the quantity (mass) of the raw material added.

A certain preferred embodiment is, for example, a zirconia composite sintered body in which the zirconia enhancer comprises TiO₂, and the TiO₂ content is 0.6 to 3.7 mass%.

A certain preferred embodiment is, for example, a zirconia composite sintered body that comprises zirconia, a stabilizer, at least one of Nb₂O₅ or Ta₂O₅, and a capping element or ion,
wherein the zirconia content is 78 to 97.5 mol%, the content of the stabilizer is 1 to 12 mol%, and the combined content of Nb₂O₅ and Ta₂O₅ is 1 to 9 mol% relative to the total moles of zirconia, the stabilizer, Nb₂O₅, and Ta₂O₅ in total 100 mol% of zirconia, the stabilizer, Nb₂O₅, and Ta₂O₅,
the stabilizer comprises Y₂O₃ and/or CeO₂,
the zirconia enhancer comprises TiO₂, and the TiO₂ content is 0.6 to 3.7 mass%,
the content of the capping element or ion is more than 0 mol% and 5 mol% or less relative to total 100 mol% of zirconia, the stabilizer, Nb₂O₅, and Ta₂O₅, and
the zirconia composite sintered body has a ratio A/B of 0.9 to 3, where A is the content of the stabilizer in mol%, and B is the combined content of Nb₂O₅ and Ta₂O₅ in mol%.

A zirconia composite sintered body of the present invention has an average crystal grain size of preferably 0.3 to 10 µm. In view of even superior machinability, strength and translucency, the average crystal grain size is more preferably 0.4 to 8 µm, even more preferably 0.5 to 5.0 µm, particularly preferably 0.5 to 4.5 µm, most preferably 1.0 to 4.0 µm. The method of measurement of average crystal grain size is as described in the EXAMPLES below.

The average crystal grain size can be measured by adjusting the particle count in one SEM image field to about 50, 100, 200, 500, or 1,000 particles, according to the method described in the EXAMPLES below.

In the zirconia composite sintered body, higher densities result in fewer internal voids, improved translucency due to less light scattering, and enhanced strength. In view of this, the density of the zirconia composite sintered body is preferably 5.5 g/cm³ or more, more preferably 5.7 g/cm³ or more, even more preferably 5.9 g/cm³ or more.

Particularly preferably, the zirconia composite sintered body is essentially void free.

The density of the composite sintered body can be calculated by dividing its mass by its volume ((mass of composite sintered body) / (volume of composite sintered body)).

The following describes a method for producing a zirconia composite sintered body of the present invention, taking as an example a zirconia composite sintered body that comprises zirconia, a stabilizer, and at least one of Nb₂O₅ or Ta₂O₅.

Zirconia composite sintered bodies of other embodiments can also be produced using the same or similar methods with the type and content of the raw material composition modified in accordance with the intended zirconia composite sintered body.

An example of a production method of a zirconia composite sintered body of the present invention is a method for producing a zirconia composite sintered body comprising the steps of fabricating a molded body using a raw material composition, and sintering the molded body.

In the production of, for example, a zirconia composite sintered body that comprises zirconia, a stabilizer capable of preventing a phase transformation of zirconia, and at least one of Nb₂O₅ or Ta₂O₅, a desired molded body can be obtained using a raw material composition comprising zirconia, the stabilizer, and at least one of Nb₂O₅ or Ta₂O₅.

In the production of, for example, a zirconia composite sintered body that comprises zirconia, the stabilizer, at least one of Nb₂O₅ or Ta₂O₅, and an element or ion derived from a capping agent, a desired molded body can be obtained using a raw material composition comprising zirconia, the stabilizer, at least one of Nb₂O₅ or Ta₂O₅, and a capping agent.

The raw material composition of the zirconia composite sintered body may be in a dry state, or in a liquid state containing liquid or contained in liquid. For example, the raw material composition may have a form of a powder, a granule or granulated material, a paste, or a slurry.

In an embodiment where the zirconia composite sintered body produced comprises a capping element or ion, the raw material composition comprises a capping agent. The capping agent is not particularly limited, as long as it is a compound with the potential to become a monovalent ion (+1 cation or -1 anion) in a solvent containing water. Examples include hydroxides, salts, halides (fluorides, chlorides, bromides, iodides), and cyanides containing elements or ions derived from a capping agent. The capping agent may be used alone, or two or more thereof may be used in combination.

Examples of hydroxides containing capping elements or ions include lithium hydroxide, sodium hydroxide, potassium hydroxide, rubidium hydroxide, cesium hydroxide, and francium hydroxide.

Examples of salts containing capping elements or ions include carbonates, bicarbonates, nitrates, hypochlorites, chlorites, chlorates, perchlorates, bromates, permanganates, metaborates, sulfides, and cyanides.

Examples of carbonates containing capping elements or ions include lithium carbonate, sodium carbonate, potassium carbonate, rubidium carbonate, francium carbonate, and cesium carbonate.

Examples of bicarbonates containing capping elements or ions include lithium bicarbonate, sodium bicarbonate, potassium bicarbonate, rubidium bicarbonate, francium bicarbonate, and cesium bicarbonate.

Examples of nitrates containing capping elements or ions include calcium nitrate, strontium nitrate, iron(II) nitrate, iron(III) nitrate, cobalt(II) nitrate, magnesium nitrate, gallium nitrate, yttrium(III) nitrate, lanthanum(III) nitrate, praseodymium nitrate, neodymium(III) nitrate, manganese(II) nitrate, europium nitrate, copper(II) nitrate, thorium nitrate, aluminum nitrate, nickel(II) nitrate, chromium(III) nitrate, titanium(IV) nitrate, zirconium nitrate, zirconium(IV) oxynitrate hydrate (ZrO(NO₃)₂·xH₂O), cerium(III) nitrate, tin nitrate, bismuth(III) nitrate, scandium(III) nitrate, indium(III) nitrate, and hafnium(IV) nitrate.

Examples of hypochlorites containing capping elements or ions include sodium hypochlorite, and calcium hypochlorite.

Examples of chlorites containing capping elements or ions include sodium chlorite, potassium chlorite, lithium chlorite, calcium chlorite, magnesium chlorite, barium chlorite, copper(II) chlorite, copper(III) chlorite, silver chlorite, and nickel chlorite.

Examples of chlorates containing capping elements or ions include calcium chlorate, barium chlorate, cobalt chlorate, nickel chlorate, magnesium chlorate, zinc chlorate, and copper chlorate.

Examples of perchlorates containing capping elements or ions include iron(III) perchlorate, barium perchlorate, calcium perchlorate, cobalt perchlorate, nickel perchlorate, magnesium perchlorate, beryllium perchlorate, aluminum perchlorate, and cerium perchlorate.

Examples of bromates containing capping elements or ions include neodymium bromate, lanthanum bromate, and praseodymium bromate.

Examples of permanganates containing capping elements or ions include calcium(VII) permanganate, potassium(VII) permanganate, and sodium(VII) permanganate.

Examples of metaborates containing capping elements or ions include sodium metaborate, and barium metaborate.

Examples of sulfides containing capping elements or ions include copper(I) sulfide.

Examples of cyanides containing capping elements or ions include barium cyanide, sodium cyanide, potassium cyanide, and calcium cyanide.

Examples of fluorides containing capping elements or ions include lithium fluoride, sodium fluoride, potassium fluoride, rubidium fluoride, cesium fluoride, francium fluoride, beryllium fluoride, magnesium fluoride, calcium fluoride, strontium fluoride, barium fluoride, scandium(III) fluoride, yttrium(III) fluoride, lanthanum(III) fluoride, cerium(III) fluoride, neodymium(III) fluoride, titanium(III) fluoride, titanium(IV) fluoride, zirconium(IV) fluoride, hafnium(IV) fluoride, tantalum(V) fluoride, manganese(II) fluoride, manganese(III) fluoride, iron(II) fluoride, iron(III) fluoride, copper(II) fluoride, zinc(II) fluoride, aluminum fluoride, chromium(III) fluoride, bismuth(III) fluoride, indium(III) fluoride, and tin(II) fluoride.

Examples of chlorides containing capping elements or ions include zirconium oxychloride, lithium chloride, sodium chloride, potassium chloride, rubidium chloride, cesium chloride, francium chloride, beryllium chloride, magnesium chloride, calcium chloride, strontium chloride, barium chloride, scandium(III) chloride, yttrium(III) chloride, lanthanum(III) chloride, cerium(III) chloride, praseodymium chloride, neodymium(III) chloride, samarium chloride, europium chloride, titanium(III) chloride, titanium(IV) chloride, zirconium(IV) chloride, hafnium(IV) chloride, tantalum(V) chloride, manganese chloride, iron(II) chloride, iron(III) chloride, cobalt(II) chloride, nickel(II) chloride, copper(I) chloride, copper(II) chloride, zinc(II) chloride, aluminum chloride, gallium chloride, bismuth(III) chloride, indium(I) chloride, indium(III) chloride, and tin(II) chloride.

Examples of bromides containing capping elements or ions include lithium bromide, sodium bromide, potassium bromide, rubidium bromide, cesium bromide, francium bromide, beryllium bromide, magnesium bromide, calcium bromide, strontium bromide, barium bromide, scandium(III) bromide, yttrium(III) bromide, cerium(III) bromide, neodymium(III) bromide, titanium(IV) bromide, zirconium(IV) bromide, tantalum(V) bromide, manganese(II) bromide, iron(II) bromide, iron(III) bromide, cobalt(II) bromide, nickel(II) bromide, copper(I) bromide, copper(II) bromide, zinc(II) bromide, chromium(III) bromide, bismuth(III) bromide, vanadium(III) bromide, indium(III) bromide, and tin bromide.

Examples of iodides containing capping elements or ions include iodides such as lithium iodide, sodium iodide, potassium iodide, rubidium iodide, cesium iodide, francium iodide, beryllium iodide, calcium iodide, magnesium iodide, strontium iodide, barium iodide, scandium(III) iodide, yttrium(III) iodide, lanthanum(III) iodide, cerium(III) iodide, neodymium(III) iodide, titanium(IV) iodide, zirconium(IV) iodide, hafnium(IV) iodide, tantalum(V) iodide, manganese(II) iodide, iron(II) iodide, iron(III) iodide, cobalt(II) iodide, nickel(II) iodide, copper(I) iodide, zinc(II) iodide, aluminum iodide, chromium(III) iodide, vanadium(II) iodide, bismuth(III) iodide, indium(III) iodide, tin(II) iodide, and tin(IV) iodide.

Commercially available zirconia powders can be used for zirconia.

Examples of such commercially available products include zirconia powders manufactured by Tosoh Corporation under the trade names Zpex^{®} (Y₂O₃ content: 3 mol%), Zpex^{®} 4 (Y₂O₃ content: 4 mol%), Zpex^{®} Smile^{®} (Y₂O₃ content: 5.5 mol%), TZ-3Y (Y₂O₃ content: 3 mol%), TZ-3YS (Y₂O₃ content: 3 mol%), TZ-4YS (Y₂O₃ content: 4 mol%), TZ-6Y (Y₂O₃ content: 6 mol%), TZ-6YS (Y₂O₃ content: 6 mol%), TZ-8YS (Y₂O₃ content: 8 mol%), TZ-10YS (Y₂O₃ content: 10 mol%), TZ-3Y-E (Y₂O₃ content: 3 mol%), TZ-3YS-E (Y₂O₃ content: 3 mol%), TZ-3YB-E (Y₂O₃ content: 3 mol%), TZ-3YSB-E (Y₂O₃ content: 3 mol%), TZ-3YB (Y₂O₃ content: 3 mol%), TZ-3YSB (Y₂O₃ content: 3 mol%), TZ-3Y20AB (Y₂O₃ content: 3 mol%), TZ-8YSB (Y₂O₃ content: 8 mol%), and TZ-0 (Y₂O₃ content: 0 mol%). These commercially available zirconia powders also contain HfO₂. It is possible to use commercially available products additionally containing Y₂O₃.

The raw material composition of the present invention may use zirconia powders in which Y₂O₃ is uniformly dispersed as a solid solution, as in the TZ series of the commercially available products listed above (those with "TZ" in the product names).

The method of production of zirconia powder is not particularly limited, and known methods may be employed, for example, such as the breakdown process, where coarse particles are pulverized into fine powder, or the building-up process, where synthesis occurs through nucleation and growth from atoms or ions.

The type of zirconia powder in the raw material composition is not particularly limited. It is possible to additionally incorporate stabilizer particles when the zirconia powder comprises zirconia but does not contain a stabilizer, or when increasing the content of stabilizer as needed. The stabilizer particles are not particularly limited, as long as the content of the stabilizer within the zirconia composite sintered body can be adjusted to the predetermined ranges mentioned above.

For example, the stabilizer particles may be commercially available products, and may be prepared by pulverizing a commercially available powder using a known pulverizing mixer (such as a ball mill).

The stabilizer may be either a stabilizer not dissolved in zirconia as a solid solution, or a stabilizer dissolved in zirconia as a solid solution.

In view of ease of obtaining the desired zirconia composite sintered body, a certain preferred embodiment is, for example, a method for producing a zirconia composite sintered body in which the stabilizer (preferably, Y₂O₃) in the raw material composition comprises a stabilizer not dissolved in zirconia as a solid solution. The presence of stabilizers not dissolved in zirconia as a solid solution can be verified, for example, through X-ray diffraction (XRD) patterns.

The presence of peaks derived from the stabilizer in an XRD pattern of the raw material composition or molded body indicates the presence of a stabilizer that is not dissolved in zirconia in the raw material composition or molded body.

A peak derived from the stabilizer is basically not observable in an XRD pattern when the stabilizer is fully dissolved in zirconia as a solid solution. It is, however, possible, depending on the crystal state or other conditions of the stabilizer, that the stabilizer is not dissolved in zirconia as a solid solution even when the XRD pattern does not show peaks for stabilizers.

The following describes situations where the stabilizer comprises a stabilizer not dissolved in zirconia as a solid solution, with yttria serving as an example of the stabilizer.

In the raw material composition or molded body of the present invention, the abundance f_{y} of yttria not dissolved in zirconia as a solid solution (hereinafter, also referred to as "undissolved yttria") can be calculated using the following mathematical formula (2).${f}_{y} = {I}_{29} / \left({I}_{28}+{I}_{29}+{I}_{30}\right) \times 100$ (2) where f_{y} represents the fraction (%) of undissolved yttria, I₂₈ represents the integrated intensity of a peak near 2θ = 28°, where the main peak of the monoclinic zirconia appears in XRD measurement, I₂₉ represents the integrated intensity of a peak near 2θ = 29°, where the main peak of yttria appears in XRD measurement, and I₃₀ represents the integrated intensity of a peak near 2θ = 30°, where the main peak of tetragonal or cubic zirconia appears in XRD measurement.

When additionally using stabilizers other than yttria, the formula can be applied to calculate the abundance of undissolved stabilizers other than yttria by substituting I₂₉ with the peaks of other stabilizers.

In view of considerations such as ease of obtaining the desired zirconia composite sintered body, the abundance f_{y} of undissolved yttria is preferably higher than 0%, more preferably 1% or more, even more preferably 2% or more, particularly preferably 3% or more. The upper limit for the abundance f_{y} of undissolved yttria may be, for example, 25% or less. Preferably, the upper limit of abundance f_{y} of undissolved yttria depends on the yttria content in the raw material composition or molded body.

For example, the abundance f_{y} of undissolved yttria falls within the following ranges when the yttria content in the raw material composition or molded body of the present invention is 3 mol% or more and 8 mol% or less.

When the yttria content is 3 mol% or more and less than 4.5 mol%, f_{y} may be 15% or less. When the yttria content is 4.5 mol% or more and less than 5.8 mol%, f_{y} may be 20% or less. When the yttria content is 5.8 mol% or more and 8 mol% or less, f_{y} may be 25% or less.

For example, when the yttria content is 3 mol% or more and less than 4.5 mol%, f_{y} is preferably 2% or more, more preferably 3% or more, even more preferably 4% or more, particularly preferably 5% or more.

When the yttria content is 4.5 mol% or more and less than 5.8 mol%, f_{y} is preferably 3% or more, more preferably 4% or more, even more preferably 5% or more, yet more preferably 6% or more, particularly preferably 7% or more.

When the yttria content is 5.8 mol% or more and 8 mol% or less, f_{y} is preferably 4% or more, more preferably 5% or more, even more preferably 6% or more, yet more preferably 7% or more, particularly preferably 8% or more.

In the raw material composition or molded body of the present invention, it is not necessarily required for the stabilizer (preferably, yttria) to be fully dissolved in zirconia as a solid solution. In the present invention, "stabilizer being dissolved as a solid solution" means that, for example, the elements (atoms, preferably yttrium atoms) contained in the stabilizer are dissolved in zirconia as a solid solution.

There is no particular restriction on Nb₂O₅ and/or Ta₂O₅ incorporated in the raw material composition of the present invention, as long as the content of Nb₂O₅ and/or Ta₂O₅ within the zirconia composite sintered body can be adjusted in the predetermined ranges mentioned above. For example, commercially available products may be used for Nb₂O₅ and/or Ta₂O₅ without any particular restrictions. These may be used after pulverizing its powder using a known pulverizing mixer (such as a ball mill).

An example method of obtaining the raw material composition involves a process whereby the raw material composition is prepared by, for example, wet mixing each raw material of the raw material composition (zirconia, a stabilizer, and optional components such as at least one of Nb₂O₅ or Ta₂O₅, a capping agent (for example, a compound with the potential to become a monovalent ion in a solvent containing water), and a zirconia enhancer) in a solvent containing water.

The method for wet mixing the raw materials in a solvent containing water is not particularly limited. For example, the raw materials may be formed into a slurry through wet pulverization and mixing using a known pulverizing mixer (such as a ball mill). The slurry can then be dried and granulated to produce a granular raw material composition.

In the wet mixing process, it is possible to additionally include additives such as binders, plasticizers, dispersants, emulsifiers, antifoaming agents, pH adjusters, and lubricants. The additives may be used alone, or two or more thereof may be used in combination.

The binder may be added to the pulverized slurry after the slurry is formed by adding a primary powder mixture of zirconia, Y₂O₃, Nb₂O₅ and/or Ta₂O₅, and, optionally, a capping agent into water.

The binder is not particularly limited, and known binders may be used. Examples of the binders include polyvinyl alcohol binders, acrylic binders, wax binders (such as paraffin wax), methyl cellulose, carboxymethyl cellulose, polyvinyl butyral, polymethylmethacrylate, ethyl cellulose, polyethylene, polypropylene, ethylene-vinyl acetate copolymer, polystyrene, atactic polypropylene, and methacrylic resin.

Examples of the plasticizers include polyethylene glycol, glycerin, propylene glycol, and dibutyl phthalic acid.

Examples of the dispersants include ammonium polycarboxylates (such as triammonium citrate), ammonium polyacrylate, acrylic copolymer resin, acrylic acid ester copolymer, polyacrylic acid, bentonite, carboxymethyl cellulose, anionic surfactants (for example, polyoxyethylene alkyl ether phosphoric acid esters such as polyoxyethylene lauryl ether phosphoric acid ester), non-ionic surfactants, oleic glyceride, amine salt surfactants, oligosaccharide alcohols, and stearic acid.

Examples of the emulsifiers include alkyl ethers, phenyl ethers, and sorbitan derivatives.

Examples of the antifoaming agents include alcohols, polyethers, silicone, and waxes.

Examples of the pH adjusters include ammonia, and ammonium salts (including ammonium hydroxides such as tetramethylammonium hydroxide).

Examples of the lubricants include polyoxyethylene alkyl ethers, and waxes.

The solvent used for wet mixing is not particularly limited, as long as it contains water. By using organic solvents, the solvent may be a mixed solvent of water and organic solvent. Alternatively, the solvent may be solely water. Examples of the organic solvents include ketone solvents such as acetone, and ethyl methyl ketone; and alcohol solvents such as ethanol, 1-propanol, 2-propanol, 2-methyl-2-propanol, glycerin, diglycerin, polyglycerin, propylene glycol, dipropylene glycol, polypropylene glycol, ethylene glycol, diethylene glycol, polyethylene glycol, polyethylene glycol monomethyl ether, 1,2-pentadiol, 1,2-hexanediol, and 1,2-octanediol. The organic solvents may be used alone, or two or more thereof may be used in combination.

The raw material composition used for the zirconia composite sintered body in the present invention may comprise components other than zirconia, Y₂O₃, Nb₂O₅, Ta₂O₅, a capping agent, and a zirconia enhancer, provided that the present invention can exhibit its effects.

Examples of such additional components include colorants (pigments and composite pigments), fluorescent agents, and SiO₂. The additional components may be used alone, or two or more thereof may be used as a mixture.

Examples of the pigments include oxides of at least one element selected from the group consisting of V, Cr, Mn, Fe, Co, Ni, Zn, Y, Zr, Sn, Sb, Bi, Ce, Pr, Sm, Eu, Gd, Tb, and Er (specifically, such as NiO, and Cr₂O₃), preferably oxides of at least one element selected from the group consisting of V, Cr, Mn, Fe, Co, Ni, Zn, Y, Zr, Sn, Sb, Bi, Ce, Pr, Sm, Eu, Gd, and Tb, more preferably oxides of at least one element selected from the group consisting of V, Cr, Mn, Fe, Co, Ni, Zn, Y, Zr, Sn, Sb, Bi, Ce, Sm, Eu, Gd, and Tb. The pigments may exclude Y₂O₃ and CeO₂.

Examples of the composite pigments include (Zr,V)O₂, Fe(Fe,Cr)₂O₄, (Ni,Co,Fe)(Fe,Cr)₂O₄·ZrSiO₄, and (Co,Zn)Al₂O₄.

Examples of the fluorescent agents include Y₂SiO₅:Ce, Y₂SiO₅:Tb, (Y,Gd,Eu)BO₃, Y₂O₃:Eu, YAG:Ce, ZnGa₂O₄:Zn, and BaMgAl₁₀O₁₇:Eu.

Following its production, the raw material composition is molded to fabricate a molded body. The molding method is not particularly limited, and known molding methods can be used (for example, such as press molding).

When producing a zirconia molded body using a method that includes press molding the raw material composition, there are no specific limitations on the press molding method, and press molding may be achieved using known press molding machines. Specific examples of press molding methods include uniaxial pressing.

The pressure in press molding is appropriately optimized according to the desired size of molded body, open porosity, biaxial flexural strength, and the particle size of raw material powder. Typically, the pressure in press molding ranges from 5 MPa to 1,000 MPa. By increasing the molding pressure in the method of production above, it is possible to achieve more effective closure of pores in the resulting molded body, leading to a lower open porosity and increasing the density of the molded body. To increase the density of the zirconia molded body obtained, cold isostatic pressing (CIP) may be applied after uniaxial pressing.

Following its production, the molded body is sintered to obtain a zirconia composite sintered body.

The sintering temperature (highest sintering temperature) for obtaining the zirconia composite sintered body is, for example, preferably 1,300°C or more, more preferably 1,350°C or more, even more preferably 1,400°C or more, yet more preferably 1,450°C or more, particularly preferably 1,500°C or more. The sintering temperature is, for example, preferably 1,680°C or less, more preferably 1,650°C or less, even more preferably 1,600°C or less. Preferably, the method for producing a zirconia composite sintered body of the present invention involves firing the molded body at a highest sintering temperature of 1,300 to 1,680°C. The highest sintering temperature is preferably a temperature in the atmosphere.

Reducing the highest sintering temperature (selecting a lower temperature range) leads to reduced heat compared to conditions with higher sintering temperatures (for example, 1,680°C). This lowers the stability of tetragonal zirconia, facilitating its transformation into monoclinic zirconia, thereby making it easier to adjust and increase the monoclinic fraction at the processed surface. In view of this, the highest sintering temperature may be 1,580°C or less, or 1,550°C or less.

A certain preferred embodiment is, for example, a method for producing a zirconia composite sintered body comprising firing the molded body at a highest sintering temperature of 1,300 to 1,580°C.

The hold time (retention time) at the highest sintering temperature is preferably 30 hours or less, more preferably 20 hours or less, even more preferably 10 hours or less, yet more preferably 5 hours or less, particularly preferably 3 hours or less, most preferably 2 hours or less, depending on the temperature. The hold time may be 25 minutes or less, 20 minutes or less, or 15 minutes or less. The hold time is preferably 1 minute or more, more preferably 5 minutes or more, even more preferably 10 minutes or more. A production method of the present invention enables the fabrication of a zirconia composite sintered body having superior flexural strength, translucency, and machinability in a manner that depends on the content of the stabilizer. The sintering time can be reduced, as long as the present invention can exhibit its effects. Shortening the sintering time can improve production efficiency and reduce energy costs.

In the method for producing a zirconia composite sintered body of the present invention, the rate of temperature increase in sintering the molded body is not particularly limited, and is preferably 0.1°C/min or more, more preferably 0.2°C/min or more, even more preferably 0.5°C/min or more. The rate of temperature increase is preferably 50°C/min or less, more preferably 30°C/min or less, even more preferably 20°C/min or less. Productivity improves when the rate of temperature increase is at or above these lower limits.

A common dental zirconia furnace can be used for the sintering process of the molded body. The dental zirconia furnace may be a commercially available product. Examples of commercially available products include Noritake KATANA^{®} F-1, F-1N, F-2 (SK Medical Electronics Co., Ltd.).

Preferably, the sintering process for the molded body includes a hot isostatic pressing (HIP) process, aside from sintering at the highest sintering temperature. The HIP process can further improve the translucency and strength of the zirconia composite sintered body.

In the following, the term "primary sintered body" is used to refer to sintered bodies obtained through sintering at the highest sintering temperature, whereas "HIP sintered body" is used to refer to sintered bodies after a HIP process.

Known hot isostatic pressing (HIP) devices can be used for HIP process.

The temperature for HIP process is not particularly limited. However, for advantages such as obtaining a high-strength and dense zirconia composite sintered body, the HIP temperature is preferably 1,200°C or more, more preferably 1,300°C or more, even more preferably 1,400°C or more. The HIP temperature is preferably 1,700°C or less, more preferably 1,650°C or less, even more preferably 1,600°C or less.

In the method for producing a zirconia composite sintered body of the present invention, the HIP pressure in the HIP process of the primary sintered body is not particularly limited. However, for advantages such as obtaining a high-strength and dense sintered body, the HIP pressure is preferably 100 MPa or more, more preferably 125 MPa or more, even more preferably 130 MPa or more. The upper limit of HIP pressure is not particularly limited, and may be, for example, 400 MPa or less, 300 MPa or less, or 200 MPa or less.

In the method for producing a zirconia composite sintered body of the present invention, the rate of temperature increase in the HIP process of the primary sintered body is not particularly limited, and is preferably 0.1°C/min or more, more preferably 0.2°C/min or more, even more preferably 0.5°C/min or more. The rate of temperature increase is preferably 50°C/min or less, more preferably 30°C/min or less, even more preferably 20°C/min or less. Productivity improves when the rate of temperature increase is at or above these lower limits.

In the method for producing a zirconia composite sintered body of the present invention, the duration of HIP in the HIP process of the primary sintered body is not particularly limited. However, for advantages such as obtaining a high-strength and dense zirconia composite sintered body, the duration of the HIP process is preferably 5 minutes or more, more preferably 10 minutes or more, even more preferably 30 minutes or more. The duration of the HIP process is preferably 10 hours or less, more preferably 6 hours or less, even more preferably 3 hours or less.

In the method for producing a zirconia composite sintered body of the present invention, the pressure medium in the HIP process of the primary sintered body is not particularly limited. However, in view of a low impact on zirconia, the pressure medium may be at least one selected from the group consisting of oxygen gas, oxygen-mixed gas, air, and inert gas (for example, such as nitrogen gas, argon gas).

When conducting HIP processing of the primary sintered body in an oxygen-mixed gas atmosphere, the oxygen concentration may be, for example, more than 0% and 20% or less, though it is not particularly limited.

When using an oxygen-mixed gas, at least one inert gas (for example, such as nitrogen gas or argon gas) may be selected as a gas other than oxygen.

In a method for producing a zirconia composite sintered body of the present invention, the HIP process may result in blackening due to oxygen deficiency when the process is carried out in a reducing atmosphere such as by using an inert gas. In order to prevent such blackening, it is preferable to include a heat treatment step (or tempering as it is also called hereinbelow), performed at 1,650°C or less in either the atmosphere or an excess oxygen atmosphere, after the HIP process. In view of the efficiency of heat treatment, the heat treatment step is carried out preferably in an excess oxygen atmosphere. Here, "excess oxygen atmosphere" means an atmosphere where the oxygen concentration exceeds that of the atmosphere. The excess oxygen atmosphere is not particularly limited, as long as the oxygen concentration is higher than 21% and 100% or less, and can be appropriately selected within this range. For example, the oxygen concentration may be 100%.

A zirconia composite sintered body of the present invention may be a primary sintered body, a HIP sintered body, or a tempered sintered body with no particular restriction, provided that the present invention can exhibit its effects.

A certain preferred embodiment is, for example, a zirconia composite sintered body that has been tempered.

The heat treatment temperature in the atmosphere or an excess oxygen atmosphere may be appropriately varied according the aesthetics of the zirconia composite sintered body (for example, the shade of dental prostheses).

In a certain preferred embodiment, in view of the aesthetics of the zirconia composite sintered body, the heat treatment temperature in the atmosphere or an excess oxygen atmosphere is preferably 1,650°C or less, more preferably 1,600°C or less, even more preferably 1,550°C or less.

In another preferred embodiment, in view of the aesthetics of the zirconia composite sintered body, the heat treatment temperature in the atmosphere or an excess oxygen atmosphere is preferably 1,400°C or less, more preferably 1,300°C or less, even more preferably 1,200°C or less.

In either embodiment, the heat treatment temperature is preferably 500°C or more, more preferably 600°C or more, even more preferably 700°C or more.

A common dental zirconia furnace can be used for the tempering process. The dental zirconia furnace may be a commercially available product. Examples of commercially available products include Noritake KATANA^{®} F-1, F-1N, F-2 (SK Medical Electronics Co., Ltd.).

A zirconia composite sintered body of the present invention exhibits excellent machinability despite being a sintered body. It is accordingly not necessary to machine it as a mill blank of a composite pre-sintered body in a semi-sintered state before sintering into a sintered body.

Nonetheless, the zirconia composite sintered body can be produced using a method whereby a molded body obtained from the raw material composition is pre-sintered to form a semi-sintered-state composite pre-sintered body, and this composite pre-sintered body, unprocessed, is machined before sintering it to form a sintered body.

A zirconia composite sintered body of the present invention comprises a predetermined fraction of monoclinic zirconia at the surface through machining, and exhibits excellent adhesive properties without sandblasting after machining. Accordingly, unlike Patent Literature 2, it is not required to irradiate the surface of the sintered body with a laser such as an ultrashort pulsed-laser. Put differently, a zirconia composite sintered body of the present invention does not need to have nanopores (open pores) at the surface.

Another certain embodiment is, for example, a method for producing a zirconia composite sintered body that comprises the steps of fabricating a molded body using the raw material composition, pre-sintering the molded body to form a zirconia composite pre-sintered body (pre-sintering step), and sintering the zirconia composite pre-sintered body.

In order to ensure block formation, the firing temperature (pre-sintering temperature) in the pre-sintering step is, for example, preferably 800°C or more, more preferably 900°C or more, even more preferably 950°C or more.

The pre-sintering temperature is, for example, preferably 1,200°C or less, more preferably 1,150°C or less, even more preferably 1,100°C or less. The preferred range of pre-sintering temperatures is, for example, 800°C to 1,200°C. It is believed that, at these pre-sintering temperatures, no significant progression of the formation of the solid solution of the stabilizer occurs in the pre-sintering step.

The zirconia composite pre-sintered body has a density of preferably 2.7 g/cm³ or more. The zirconia composite pre-sintered body has a density of preferably 4.0 g/cm³ or less, more preferably 3.8 g/cm³ or less, even more preferably 3.6 g/cm³ or less. Processing becomes easier when the density falls within these ranges. The density of the composite pre-sintered body can be calculated, for example, as the mass-to-volume ratio of the composite pre-sintered body ((mass of composite pre-sintered body) / (volume of composite pre-sintered body)).

The zirconia composite pre-sintered body has a three-point flexural strength of preferably 15 to 70 MPa, more preferably 18 to 60 MPa, even more preferably 20 to 50 MPa.

The flexural strength can be measured in compliance with ISO 6872:2015 except for the specimen size, using a specimen measuring 5 mm in thickness, 10 mm in width, and 50 mm in length. For surface finishing, the specimen surfaces, including chamfered surfaces (45° chamfers at the corners of specimen), are finished longitudinally with #600 sandpaper. The specimen is disposed in such an orientation that its widest face is perpendicular to the vertical direction (loading direction). In the flexure test, measurements are made at a span of 30 mm with a crosshead speed of 1.0 mm/min.

The sintering step for the zirconia composite pre-sintered body can be conducted using the same method and conditions (such as temperature and pressure) used to sinter the molded body. Accordingly, in embodiments of the production method using a zirconia composite pre-sintered body, the term "molded body" can be read as referring to "composite pre-sintered body".

A zirconia composite sintered body of the present invention excels in strength. A zirconia composite sintered body of the present invention has a biaxial flexural strength of preferably 300 MPa or more, more preferably 350 MPa or more, even more preferably 400 MPa or more, yet more preferably 450 MPa or more, particularly preferably 500 MPa or more. A zirconia composite sintered body of the present invention, with its biaxial flexural strength falling in these ranges, can reduce defects, for example, such as fracture in the oral cavity, when used as a dental prosthesis. The upper limit of biaxial flexural strength is not particularly limited, and may be, for example, 1,200 MPa or less, or 1,000 MPa or less. The biaxial flexural strength of the zirconia composite sintered body can be measured in compliance with ISO 6872:2015.

It is preferable that a zirconia composite sintered body of the present invention have high translucency. The translucency can be evaluated using ΔL*(W-B).

Specifically, a zirconia composite sintered body of the present invention has a translucency of preferably 10 or more, more preferably 11 or more, even more preferably 12 or more, particularly preferably 13 or more in terms of a ΔL*(W-B) in a sample measuring 15 mm in diameter and 1.2 mm in thickness.

A highly aesthetic zirconia composite sintered body more closely resembling natural teeth can be obtained with ΔL*(W-B) falling within these ranges.

Here, ΔL*(W-B) means the difference between the lightness (first L* value) against a white background and the lightness (second L* value) against a black background in the same sample. Specifically, ΔL*(W-B) means the difference between the L* value against a white background (JIS Z 8781-4:2013 Color Measurements - Part 4: CIE 1976 L*a*b* color space) and the L* value against a black background. The white background means the white part of the hiding-power test paper described in JIS K 5600-4-1:1999, Part 4, Section 1, and the black background means the black part of the hiding-power test paper.

The upper limit of ΔL*(W-B) is not particularly limited, and may be, for example, 25 or less. In view of aesthetics, ΔL*(W-B) may be 20 or less.

A spectrophotometer can be used to measure ΔL* for a zirconia composite sintered body with a diameter of 15 mm and a thickness of 1.2 mm. For example, the dental colorimeter Crystaleye CE100-CE/JP with a 7-band LED illuminant and analysis software Crystaleye (manufactured by Olympus Corporation) may be used for measurement.

Examples of dental prostheses produced using a zirconia composite sintered body of the present invention include crown restorations such as inlays, onlays, veneers, crowns, core-integrated crowns, and bridges, as well as abutment teeth, dental posts, dentures, denture bases, and implant parts (fixtures, abutments).

Preferably, for example, a commercially available dental CAD/CAM system is used for machining. Examples of such CAD/CAM systems include the CEREC system manufactured by Dentsply Sirona, and the KATANA^{®} system manufactured by Kuraray Noritake Dental Inc.

A zirconia composite sintered body of the present invention can also be used in applications other than dental use, and is particularly suited for zirconia parts that require irregular or complex shapes and strength.

Compared to sintered bodies produced solely by existing production methods (such as injection molding, CIP, casting, and 3D printing), a zirconia composite sintered body of the present invention can be directly processed as a sintered body. This can be cost-effective, for example, when a desired zirconia part is obtained in short time periods. For parts that are complex in shape and difficult to produce with traditional methods, this technique eliminates the need for mechanical fitting of multiple parts, allowing for the production of zirconia parts with maintained high strength. Furthermore, because the sintered body is directly processable, the sintering process can be eliminated when precise dimensions are needed, eliminating uneven shrinkage during firing, leading to high-precision zirconia parts. A zirconia composite sintered body of the present invention is also applicable to methods of manufacture in applications, specifically, such as in jewelry goods, engine parts and interior components of mobility vehicles such as aircraft and automobiles, display panel frames, construction members, electrical appliance components, components of household goods, and toy parts.

The zirconia parts can also be fitted with different materials to create composite components.

Another embodiment is, for example, a dental kit for conservative restoration comprising any of the dental zirconia composite sintered bodies above in combination with a dental cement.

The dental cement is not particularly limited, and may be a known dental cement. Examples of known dental cements can be found in, for example, JP2002-212019 A, JP2013-209341 A, and JP2017-105715 A.

The dental cement is preferably one comprising an acidic component.

In view of high adhesive properties exhibited when combined with Nb₂O₅ and/or Ta₂O₅, and, optionally, the capping element contained in the dental zirconia composite sintered body, the acidic component is preferably one comprising a monomer having an acidic group. The monomer having an acidic group may be used alone, or two or more thereof may be used in combination.

The monomer having an acidic group is preferably one comprising at least one selected from the group consisting of a phosphoric acid group, a pyrophosphoric acid group, a thiophosphoric acid group, a phosphonic acid group, a carboxylic acid group, and a sulfonic acid group.

The monomer having an acidic group is preferably a (meth)acrylic monomer having at least one polymerizable group such as a (meth)acryloyloxy group and a (meth)acrylamide group.

Examples of the phosphoric acid group-containing (meth)acrylic monomer include:
phosphoric acid group-containing monofunctional (meth)acrylate monomers, including, for example, phosphoric acid group-containing monofunctional (meth)acrylate compounds such as 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyeicosyl dihydrogen phosphate, 2-(meth)acryloyloxyethylphenyl hydrogen phosphate, 2-(meth)acryloyloxyethyl-2-bromoethyl hydrogen phosphate, 2-(meth)acryloyloxyethyl-(4-methoxyphenyl) hydrogen phosphate, and 2-(meth)acryloyloxypropyl-(4-methoxyphenyl) hydrogen phosphate, and acid chlorides, alkali metal salts, ammonium salts, and amine salts thereof; and
phosphoric acid group-containing bifunctional (meth)acrylate monomers, including, for example, phosphoric acid group-containing bifunctional (meth)acrylate compounds such as bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis[8-(meth)acryloyloxyoctyl] hydrogen phosphate, bis[9-(meth)acryloyloxynonyl] hydrogen phosphate, bis[10-(meth)acryloyloxydecyl] hydrogen phosphate, and 1,3-di(meth)acryloyloxypropyl dihydrogen phosphate, and acid chlorides, alkali metal salts, ammonium salts, and amine salts thereof.

Examples of the pyrophosphoric acid group-containing (meth)acrylic monomer include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, and bis[10-(meth)acryloyloxydecyl] pyrophosphate, and acid chlorides, alkali metal salts, ammonium salts, and amine salts thereof.

Examples of the thiophosphoric acid group-containing (meth)acrylic monomer include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, and 20-(meth)acryloyloxyeicosyl dihydrogen thiophosphate, and acid anhydrides, acid halides (such as acid chlorides), alkali metal salts, and ammonium salts thereof.

Examples of the phosphonic acid group-containing (meth)acrylic monomer include 2-(meth)acryloyloxyethyl phenyl phosphonate, 5-(meth)acryloyloxypentyl 3-phosphonopropionate, 6-(meth)acryloyloxyhexyl 3-phosphonopropionate, 10-(meth)acryloyloxydecyl 3-phosphonopropionate, 6-(meth)acryloyloxyhexyl phosphonoacetate, and 10-(meth)acryloyloxydecyl phosphonoacetate, and acid chlorides, alkali metal salts, ammonium salts, and amine salts thereof.

Examples of the carboxylic acid group-containing (meth)acrylic monomer include:
(meth)acrylic acid, 4-[2-[(meth)acryloyloxy]ethoxycarbonyl] phthalic acid, 4-(meth)acryloyloxyethyl trimellitic acid, 4-(meth)acryloyloxybutyl oxycarbonyl phthalic acid, 4-(meth)acryloyloxyhexyl oxycarbonyl phthalic acid, 4-(meth)acryloyloxyoctyl oxycarbonyl phthalic acid, and 4-(meth)acryloyloxydecyl oxycarbonyl phthalic acid, and acid anhydrides thereof; and
5-(meth)acryloylaminopentylcarboxylic acid, 6-(meth)acryloyloxyhexane-1,1-dicarboxylic acid, 8-(meth)acryloyloxyoctane-1,1-dicarboxylic acid, 10-(meth)acryloyloxydecane-1,1-dicarboxylic acid, and 11-(meth)acryloyloxyundecane-1,1-dicarboxylic acid, and acid chlorides, alkali metal salts, ammonium salts, and amine salts thereof.

Examples of the sulfonic acid group-containing (meth)acrylic monomer include 2-(meth)acrylamido-2-methylpropanesulfonic acid, and 2-sulfoethyl (meth)acrylate, and acid chlorides, alkali metal salts, ammonium salts, and amine salts thereof.

Preferred among these monomers having an acidic group are phosphoric acid group-containing (meth)acrylic monomers, pyrophosphoric acid group-containing (meth)acrylic monomers, and carboxylic acid group-containing (meth)acrylic monomers for their ability to exhibit even superior adhesive strength. More preferred are phosphoric acid group-containing (meth)acrylic monomers, and carboxylic acid group-containing (meth)acrylic monomers, even more preferably phosphoric acid group-containing monofunctional (meth)acrylate monomers.

Among these monomers, more preferred are phosphoric acid group-containing monofunctional (meth)acrylate monomers having a C₆ to C₂₀ alkyl group or C₆ to C₂₀ alkylene group as the main chain within the molecule, and carboxylic acid group-containing (meth)acrylate monomers such as 4-(meth)acryloyloxyethyltrimellitic acid, and 4-(meth)acryloyloxyethyltrimellitic acid anhydride, most preferably phosphoric acid group-containing monofunctional (meth)acrylate monomers having a C₈ to C₁₂ alkylene group as the main chain within the molecule, such as 10-methacryloyloxydecyl dihydrogen phosphate.

The dental cement may comprise a hydrophobic crosslinkable monomer having no acidic group (hereinafter, also referred to simply as "hydrophobic crosslinkable monomer"), other than the monomer having an acidic group.

The hydrophobic crosslinkable monomer is a hydrophobic compound having at least two polymerizable groups (such as (meth)acryloyloxy groups, and (meth)acrylamide groups), without containing an acidic group within the molecule. Here, "hydrophobic" means a solubility of less than 5 mass% in water at 25°C.

The hydrophobic crosslinkable monomer has the effect of improving the handling properties and mechanical strength of a dental cement according to the present invention.

Examples of the hydrophobic crosslinkable monomer include aromatic bifunctional polymerizable monomers, aliphatic bifunctional polymerizable monomers, and tri- and higher-functional polymerizable monomers. The hydrophobic crosslinkable monomer may be used alone, or two or more thereof may be used in combination.

Examples of the aromatic bifunctional polymerizable monomers include bifunctional (meth)acrylate compounds such as 2,2-bis((meth)acryloyloxyphenyl)propane, 2,2-bis[4-(2-hydroxy-3-(meth)acryloyloxypropoxy)phenyl]propane, 2,2-bis(4-(meth)acryloyloxyethoxyphenyl)propane, 2,2-bis(4-(meth)acryloyloxypolyethoxyphenyl)propane (with an average of 2.6 moles of ethoxy group added), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane.

Examples of the aliphatic bifunctional polymerizable monomers include bifunctional (meth)acrylate compounds such as glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 2,2,4-trimethylhexamethylenebis(2-carbamoyloxyethyl) di(meth)acrylate, and 1,2-bis(3-(meth)acryloyloxy-2-hydroxypropoxy)ethane.

Examples of the tri- and higher-functional polymerizable monomers include tri- and higher-functional (meth)acrylate compounds such as trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N'-(2,2,4-trimethylhexamethylene)bis[2-(aminocarboxy)propane-1,3-diol] tetra(meth)acrylate, and 1,7-diacryloyloxy-2,2,6,6-tetra(meth)acryloyloxymethyl-4-oxaheptane.

Preferably, the dental cement comprises a chemical polymerization initiator.

The chemical polymerization initiator may be selected from polymerization initiators used in industry. Particularly preferred for use are chemical polymerization initiators used for dental cements.

The chemical polymerization initiator used in the present invention comprises an oxidizing agent and a reducing agent.

Examples of the oxidizing agent of the chemical polymerization initiator include organic peroxides, azo compounds, and inorganic peroxides. The oxidizing agent may be used alone, or two or more thereof may be used in combination.

Examples of the organic peroxides include diacyl peroxides, peroxyesters, dialkyl peroxides, peroxyketals, ketone peroxides, and hydroperoxides.

Specific examples of diacyl peroxides include benzoyl peroxide, 2,4-dichlorobenzoyl peroxide, and m-toluoyl peroxide.

Specific examples of peroxyesters include t-butyl peroxybenzoate, bis(t-butylperoxy)isophthalate, 2,5-dimethyl-2,5-bis(benzoylperoxy)hexane, t-butyl peroxy-2-ethylhexanoate, and t-butyl peroxyisopropyl carbonate.

Specific examples of dialkyl peroxides include dicumyl peroxide, di-t-butyl peroxide, and lauroyl peroxide.

Specific examples of peroxyketals include 1,1-bis(t-butylperoxy)3,3,5-trimethylcyclohexane, 1,1-bis(t-butylperoxy)cyclohexane, and 1,1-bis(t-hexylperoxy)cyclohexane.

Specific examples of ketone peroxides include methyl ethyl ketone peroxide, cyclohexanone peroxide, and methyl acetoacetate peroxide.

Specific examples of hydroperoxides include t-butyl hydroperoxide, cumene hydroperoxide, p-diisopropylbenzene hydroperoxide, and 1,1,3,3-tetramethylbutyl hydroperoxide.

Examples of azo compounds include 2,2'-azobisisobutyronitrile, and azobisisobutyl valeronitrile.

Examples of inorganic peroxides include sodium persulfate, potassium persulfate, aluminum persulfate, and ammonium persulfate.

Examples of the reducing agent of the chemical polymerization initiator include aromatic amines with no electron withdrawing group on the aromatic ring, as well as thiourea compounds, and ascorbic acid. The reducing agent may be used alone, or two or more thereof may be used in combination.

Examples of aromatic amines with no electron withdrawing group on the aromatic ring include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, and N,N-dimethyl-3,5-di-t-butylaniline.

The aromatic amines with no electron withdrawing group on the aromatic ring may be used alone, or two or more thereof may be used in combination.

Examples of thiourea compounds include thiourea, methylthiourea, ethylthiourea, ethylenethiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 1-(2-pyridyl)-2-thiourea, and 4,4-dimethylethylenethiourea. The thiourea compounds may be used alone, or two or more thereof may be used in combination.

In view of the curability of the composition, the preferred combinations of the oxidizing agent and the reducing agent are combinations of hydroperoxides (oxidizing agent) and thiourea compounds (reducing agent), and combinations of diacyl peroxides and/or inorganic peroxide (oxidizing agent) and aromatic amines with no electron withdrawing group on the aromatic ring (reducing agent).

In order to provide a dual-cure composition that also undergoes polymerization upon irradiation with light, the dental cement may comprise a known photopolymerization initiator, in addition to the chemical polymerization initiator.

Examples of such photopolymerization initiators include photopolymerization initiators applicable to dental cements, including, for example, (bis)acylphosphine oxides, water-soluble acylphosphine oxides, thioxanthones or quaternary ammonium salts of thioxanthones, ketals, α-diketones, coumarins, anthraquinones, benzoin alkyl ether compounds, and α-aminoketone compounds. The photopolymerization initiator may be used alone, or two or more thereof may be used in combination.

Preferred among these is at least one selected from the group consisting of a (bis)acylphosphine oxide and an α-diketone. Particularly preferably, the (bis)acylphosphine oxide is 2,4,6-trimethylbenzoyldiphenylphosphine oxide, and the α-diketone is camphorquinone.

In the dental cement, the chemical polymerization initiator and/or photopolymerization initiator are used preferably with a polymerization accelerator.

Examples of the polymerization accelerator include aliphatic amines, aromatic tertiary amines having an electron withdrawing group, sulfinic acid and salts thereof, sulfur-containing reducing inorganic compounds, borate compounds, derivatives of barbituric acid, triazine compounds, copper compounds, tin compounds, vanadium compounds, halogen compounds, aldehydes, and thiol compounds. The polymerization accelerator may be used alone, or two or more thereof may be used in combination.

Preferably, the dental cement comprises a filler.

Examples of the filler include organic fillers, inorganic fillers, and organic-inorganic composite fillers. The filler may be used alone, or two or more thereof may be used in combination.

Examples of organic filler materials include polymethyl methacrylate, polyethyl methacrylate, a methyl methacrylate-ethyl methacrylate copolymer, crosslinked polymethyl methacrylate, crosslinked polyethyl methacrylate, polyesters, polyamides, polycarbonates, polyphenylene ethers, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used in combination. There is no particular restriction on the shape of organic filler, and the particle diameter of the filler may be appropriately selected for use.

Examples of inorganic filler materials include quartz, silica, alumina, silica-titania, silica-titania-barium oxide, silica-zirconia, silica-alumina, lanthanum glass, borosilicate glass, soda glass, barium glass, strontium glass, glass-ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. These may be used alone, or two or more thereof may be used in combination. There is no particular restriction on the shape of inorganic filler, and the inorganic filler may be appropriately selected from inorganic fillers of different shapes, such as irregularly shaped fillers and spherical fillers.

The organic-inorganic composite fillers may be those produced using the organic fillers and inorganic fillers following known methods.

The present invention encompasses embodiments combining all or part of the foregoing features and embodiments in various forms, provided that the present invention can exhibit its effects with such combinations within the technical idea of the present invention.

### EXAMPLES

The present invention is described below in greater detail through Examples. It is to be noted, however, that the present invention is in no way limited by the following Examples, and various modifications may be made by a person with ordinary skill in the art within the technical idea of the present invention.

### [Examples 1 to 9]

The measurement samples for Examples were prepared through processes including the fabrication a granular raw material composition, a molded body, and a zirconia composite sintered body (the fabrication of a primary sintered body, as well as HIP processing and tempering), and the preparation of desired shapes using a wet processing machine. Examples 2 to 9 followed the method of Example 1 using the procedures provided below, except that these were carried out under the conditions indicated in Table 1.

### [Fabrication of Granular Raw Material Composition]

To fabricate a granular raw material composition for each Example, a mixture was prepared by combining commercially available powders of ZrO₂, Y₂O₃, Nb₂O₅, and Ta₂O₅, along with a capping agent and a TiO₂ powder, so as to yield, after sintering, a zirconia composite sintered body with the content of each component indicated in Table 1. Subsequently, water was added to prepare a slurry, and the slurry was pulverized by wet ball milling until it had an average particle diameter of 0.13 µm or less. After adding a binder to the pulverized slurry, the slurry was dried with a spray dryer to obtain a granular raw material composition (hereinafter, also referred to simply as "raw material composition"). This raw material composition was used to produce a molded body, as detailed below.

The average particle diameter was measured by volume with ultrasonic waves being applied after the slurry diluted with water was subjected to 30 minutes of ultrasonication, using a laser diffraction/scattering particle size distribution analyzer (Partica LA-950 manufactured by Horiba Ltd.).

### [Fabrication of Molded Body]

For each Example, block-shaped molded bodies were prepared for the fabrication of zirconia composite sintered body samples intended for the evaluation of degree of penetration, surface roughness, surface irregularities, and adhesive properties, as follows.

To prepare a block-shaped molded body, the raw material composition was placed in a mold with inside dimensions of 19 mm × 18 mm, ensuring that sintering produces a zirconia composite sintered body with a height of 14.5 mm.

Subsequently, the raw material composition was molded under a surface pressure of 200 MPa for 90 seconds to obtain a block-shaped molded body, using a uniaxial press molding machine.

### [Fabrication of Primary Sintered Body]

The block-shaped molded body was held for 2 hours in the atmosphere at the highest sintering temperature specified in Table 1, using the Noritake KATANA^{®} F-1 furnace manufactured by SK Medical Electronics Co., Ltd. This resulted in a block-shaped sample of a zirconia composite sintered body (primary sintered body).

### [Fabrication of HIP Sintered Body]

The block-shaped zirconia composite sintered body (primary sintered body) was held for 2 hours at 150 MPa in an argon atmosphere at the HIP temperature specified in Table 1, using the HIP device O₂-Dr.HIP manufactured by Kobe Steel, Ltd. This resulted in a block-shaped sample of a zirconia composite sintered body (HIP sintered body).

### [Fabrication of Zirconia Composite Sintered Body (Tempered Sintered Body)]

The block-shaped zirconia composite sintered body (HIP sintered body) was held at 700°C for 60 hours using the Noritake KATANA^{®} F-1 furnace (manufactured by SK Medical Electronics Co., Ltd.) to prepare a block-shaped sample of a zirconia composite sintered body (tempered sintered body). The block-shaped sample measured 15.7 mm in width, 16.5 mm in length, and 14.5 mm in height.

### [Preparation of Desired Shapes Using Wet Processing Machine]

A metal fixture was attached to the surface of the block-shaped sample of the zirconia composite sintered body (tempered sintered body) fabricated in each Example (the surface measuring 15.7 mm in width and 14.5 mm in height). Using a CEREC system MC-XL (manufactured by Dentsply Sirona), the sintered body was processed into a sample for the evaluation of degree of liquid penetration (4 mm in width × 4 mm in length × 10 mm in height), a sample for the evaluation of surface roughness Ra (10 mm in diameter × 5 mm in thickness), a sample for the evaluation of mean spacing of irregularities RSm (10 mm in diameter × 5 mm in thickness), and a sample for the evaluation of adhesive properties (10 mm in diameter × 5 mm in thickness). For the machining program, inLab^{®} CAM version 20.0.1.203841 software was used with the following settings: Manufacture: IVOCLAR VIVADENT, Material name: IPS e.max CAD, Production Method: Grinding, Block size: C16. A Step Bur 12 and a Cylinder Pointed Bur 12S were used as the processing tools.

In Example 1 and Examples 3 to 9, the machined samples of the sintered bodies were used for evaluations. In Example 2, the machined samples of the sintered bodies were evaluated after sandblasting with an Alumina Abrasive 50 Micron manufactured by J. Morita Corp.

### [Comparative Examples 1 and 2]

The measurement samples for Comparative Examples were prepared through processes including the fabrication a granular raw material composition, a molded body, and a zirconia composite pre-sintered body, and the preparation of desired shapes using a dry processing machine, as well as the fabrication of a zirconia composite sintered body.

### [Fabrication of Granular Raw Material Composition]

To fabricate a granular raw material composition for each Example, a mixture was prepared by combining commercially available powders of ZrO₂ and Y₂O₃ so as to yield, after sintering, a zirconia composite sintered body with the content of each component indicated in Table 1. Subsequently, water was added to prepare a slurry, and the slurry was pulverized by wet ball milling until it had an average particle diameter of 0.13 µm or less. After adding a binder to the pulverized slurry, the slurry was dried with a spray dryer to obtain a granular raw material composition (hereinafter, also referred to simply as "raw material composition"). This raw material composition was used to produce a molded body, as detailed below.

The average particle diameter was measured by volume with ultrasonic waves being applied after the slurry diluted with water was subjected to 30 minutes of ultrasonication, using a laser diffraction/scattering particle size distribution analyzer (Partica LA-950 manufactured by Horiba Ltd.).

### [Fabrication of Molded Body]

For each Example, a block-shaped molded body was prepared for the fabrication of a zirconia composite sintered body sample intended for the evaluation of degree of penetration, surface roughness Ra, mean spacing of irregularities RSm, and adhesive properties, as follows.

To prepare a block-shaped molded body, the raw material composition was placed in a mold with inside dimensions of 19 mm × 18 mm, ensuring that sintering produces a zirconia composite sintered body with a height of 14.5 mm.

Subsequently, the raw material composition was molded under a surface pressure of 200 MPa for 90 seconds to obtain a block-shaped molded body, using a uniaxial press molding machine.

### [Fabrication of Zirconia Composite Pre-Sintered Body]

The molded body was held at 1,100°C for 2 hours in the atmosphere, using the Noritake KATANA^{®} F-1 furnace manufactured by SK Medical Electronics Co., Ltd. This resulted in a zirconia composite pre-sintered body.

### [Preparation of Desired Shapes Using Dry Processing Machine]

Using a CEREC system MC-XL (manufactured by Dentsply Sirona), the zirconia composite pre-sintered body was processed into a sample workpiece to be sintered into a composite sintered body for the evaluation of degree of liquid penetration (4 mm in width × 4 mm in length × 10 mm in height), a sample workpiece to be sintered into a sintered body for the evaluation of surface roughness Ra (10 mm in diameter × 5 mm in thickness), a sample workpiece to be sintered into a zirconia composite sintered body for the evaluation of mean spacing of irregularities RSm (10 mm in diameter × 5 mm in thickness), and a sample workpiece to be sintered into a composite sintered body for the evaluation of adhesive properties (10 mm in diameter × 5 mm in thickness). For the machining program, inLab^{®} CAM version 20.0.1.203841 software was used with the following settings: Manufacture: KURARAY NORITAKE, Material name: KATANA Zr ONE, Production Method: Milling, Block size: CROWN. A Shaper 25 and a Finisher 10 were used as the processing tools.

### [Fabrication of Zirconia Composite Sintered Body]

The workpieces prepared were held for 2 hours in the atmosphere at the highest sintering temperature specified in Table 1, using the Noritake KATANA^{®} F-1 furnace (manufactured by SK Medical Electronics Co., Ltd.). This resulted in composite sintered bodies.

Comparative Example 1 employed the composite sintered bodies themselves as evaluation samples. In Comparative Example 2, the composite sintered bodies were used as samples in evaluations after sandblasting with an Alumina Abrasive 50 Micron manufactured by J. Morita Corp.

The content of each component in the zirconia composite sintered bodies presented in Table 1 was calculated from the quantities of the raw materials used.

The content (mol%) of capping elements or ions (e.g., Na) in Table 1 refers to the proportion external to total 100 mol% of zirconia (ZrO₂ and HfO₂ combined), the stabilizer (yttria), Nb₂O₅, and Ta₂O₅.

The content (mol%) of zirconia, the stabilizer, Nb₂O₅, and Ta₂O₅ in Table 1 refers to the content of each component in total 100 mol% of zirconia, the stabilizer, Nb₂O₅, and Ta₂O₅.

The content (mass%) of TiO₂ in Table 1 refers to the proportion external to total 100 mass% of zirconia, the stabilizer, Nb₂O₅, and Ta₂O₅.

A/B in Table 1 represents the ratio of A to B, where A is the Y₂O₃ content in mol%, and B is the combined content of Nb₂O₅ and Ta₂O₅ in mol%.

### [Measurement Method for Monoclinic Abundance fₘ of Zirconia Composite Sintered Body]

The monoclinic abundance fₘ of the present invention was determined by analyzing the crystalline phases of the zirconia composite sintered body.

Specifically, X-ray diffraction (XRD) measurement was conducted under the conditions below, using Automated Horizontal Multipurpose X-Ray Diffractometer (SmartLab, manufactured by Rigaku Corporation) and X-Ray Analysis Integrated Software (SmartLab Studio II, manufactured by Rigaku Corporation). In the measurement, the integrated intensities of the respective peaks (peak integrated intensities I) were determined, and the monoclinic abundance fₘ at the surface was calculated (arithmetic mean value for n = 2).
X-ray source: Cu Kα (λ = 1.54186 Å)
Goniometer length: 300 mm
Optical system: focusing technique
Detector: high-speed one-dimensional X-ray detector (D/teX Ultra 250)
Monochromatization: Kβ filter
Tube voltage: 45 kV
Tube current: 200 mA
Scan axis: 2θ/θ
Scan speed: 50.00°/min
Sampling step: 0.01°

The monoclinic abundance fₘ was calculated by attributing each peak to its corresponding crystalline phase, using the following formula (1). The calculated values are presented in Table 1. ${f}_{m} = {l}_{28} / \left({l}_{28}+{l}_{30}\right) \times 100$ (1)
wherein fₘ represents the monoclinic abundance (%), I₂₈ represents the integrated intensity of the peak near 2θ = 28°, where the main peak of the monoclinic zirconia appears in XRD measurement, and I₃₀ represents the integrated intensity of the peak near 2θ = 30°, where the main peak of tetragonal or cubic zirconia appears in XRD measurement.

The measurement was conducted using the disc-shaped zirconia composite sintered bodies of Examples and Comparative Examples as samples.

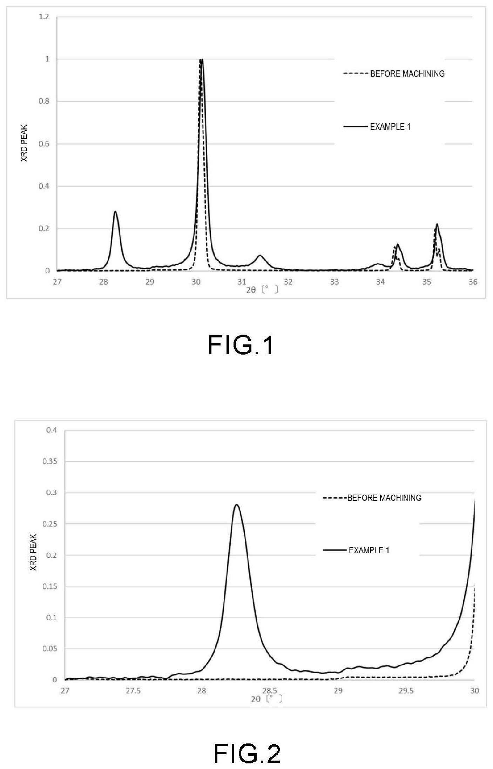

FIG. 1 represents the XRD measurement results for the dental zirconia composite sintered body according to Example 1 before and after machining. FIG. 2 is a partially enlarged view of FIG. 1.

As can be seen in FIGS. 1 and 2, the dental zirconia composite sintered body according to Example 1 was shown to include monoclinic zirconia after machining.

### [Measurement Method for Average Crystal Grain Size of Sintered Body]

Surface images were captured for the pellet-shaped zirconia composite sintered body of each Example and Comparative Example, using a scanning electron microscope (VE-9800 manufactured by Keyence under this trade name). The average crystal grain size was calculated by image analysis after indicating grain boundaries on individual crystal grains in the image.

For the measurement of average crystal grain size, the captured SEM image was binarized with image analysis software (Image-Pro Plus manufactured by Hakuto Co., Ltd. under this trade name), and particles were recognized from the field (region) by adjusting the brightness range to provide clear grain boundaries. The crystal grain size from Image-Pro Plus is the average of the measurements of the length of a line segment connecting the contour line and passing through the center of gravity determined from the contour line of the crystal grain, conducted at 2-degree intervals with the center of gravity as the central point. The measurement of crystal grain size was conducted for all particles not lying on the edges of the SEM photographic image (3 fields) of each Example and Comparative Example. The arithmetic average of the measured crystal grain sizes was then determined as the average crystal grain size (number-based) of the zirconia composite sintered body.

Note that "particles not lying on the edges of the image" refers to particles excluding those with contour lines extending beyond the frame of the SEM photographic image (particles with their contour lines interrupted by the boundary lines at the top, bottom, left, and right). To select the crystal grain size of all particles not lying on the edges of the image, the option in Image-Pro Plus was used that excludes all particles lying on the boundary lines.

### [Measurement Method for Degree of Liquid Penetration of Zirconia Composite Sintered Body]

The zirconia composite sintered body of each Example and Comparative Example was evaluated for degree of liquid penetration, using its samples (4 mm × 4 mm × 10 mm) prepared for the evaluation of degree of liquid penetration, specifically as follows.

Using a wide-mouth liquid-impermeable glass container with a bottom diameter of 5 mm or more, a sample prepared from the zirconia composite sintered body for the evaluation of degree of liquid penetration was placed upright on the bottom center of the container, with a 4 mm × 4 mm surface as the base. Subsequently, a staining solution (for example, Crack Finder manufactured by Kuraray Noritake Dental Inc. under this trade name) was added up to a vertical height of 1 mm from the base of the evaluation sample. After being allowed to stand for 2 minutes, the sample was withdrawn vertically relative to the base of the container, and rinsed with purified water to remove the staining solution. After rinsing, the length of the remaining stained region from the base of the evaluation sample was measured (arithmetic mean value for n = 5).

### [Measurement Method for Surface Roughness Ra of Zirconia Composite Sintered Body]

The zirconia composite sintered body of each Example and Comparative Example was evaluated for surface roughness Ra, using its samples (10 mm in diameter × 5 mm in thickness) prepared for the evaluation of surface roughness Ra, specifically as follows.

For the zirconia composite sintered body of each Example and Comparative Example, the surface was examined using a color 3D laser microscope (VK-9710 manufactured by Keyence Corporation under this trade name) under the following conditions.
Measurement pitch: 2 µm
Measurement distance: 1,058 µm

The acquired image was analyzed with image analysis software (VK Analyzer manufactured by Keyence Corporation under this trade name) to determine surface roughness, and the surface roughness Ra was calculated (arithmetic mean value for n = 3).

### [Measurement Method for Surface Mean Spacing of Irregularities RSm of Zirconia Composite Sintered Body]

The zirconia composite sintered body of each Example and Comparative Example was evaluated for mean spacing of irregularities RSm, using its samples (10 mm in diameter × 5 mm in thickness) prepared for the evaluation of mean spacing of irregularities RSm, specifically as follows.

For the zirconia composite sintered body of each Example and Comparative Example, the surface was examined using a color 3D laser microscope (VK-9710 manufactured by Keyence Corporation under this trade name) under the following conditions.
Measurement pitch: 2 µm
Measurement distance: 1,058 µm

The acquired image was analyzed with image analysis software (VK Analyzer manufactured by Keyence Corporation under this trade name) to determine mean spacing of irregularities, and the mean spacing of irregularities RSm was calculated (arithmetic mean value for n = 3).

### [Evaluation of Adhesive Properties of Zirconia Composite Sintered Body]

The zirconia composite sintered body of each Example and Comparative Example was evaluated for adhesive properties, using its samples (10 mm in diameter × 5 mm in thickness) prepared for the evaluation of adhesive properties, specifically as follows.

The zirconia composite sintered body sample of each Example and Comparative Example was embedded in a stainless-steel ring with a dental composite resin so as to expose the surface as indicated in Table 1 (either a machined surface or a sandblasted surface). Subsequently, a dental cement (SA Luting Multi manufactured by Kuraray Noritake Dental Inc. under this trade name) paste was applied to one end face (circular cross section) of a stainless-steel cylindrical tip (5 mm in diameter and 1.0 cm in length), and this face with the dental cement paste was placed on the embedded sample. After applying a vertical load of 500 g from above the stainless-steel cylindrical tip, the margin area between the stainless-steel cylindrical tip and the smoothed surface was irradiated with light for 2 to 5 seconds using a dental photoirradiator for polymerization (PenCure 2000 manufactured by J. Morita Corp. under this trade name), and the excess cement was removed. This was followed by 10 seconds of photoirradiation using the same photoirradiator. After 10 minutes, the sample was released from the 500 g load and used as a test sample. A total of ten test samples were prepared.

The test sample was immersed in 37°C water for 24 hours and the shear bond strength was examined. The shear bond strength was measured using a universal testing machine (AG-I 100kN manufactured by Shimadzu Corporation under this trade name) at a crosshead speed of 1 mm/min.

The arithmetic mean of the measurements for five of the ten test specimens was determined as the shear bond strength. The shear bond strength measured after immersion in 37°C water for 24 hours is presented in Table 1 as "Adhesive properties after 1 day at 37°C", X (unit: MPa).

The remaining five test specimens, which were bonded to dentin, were subjected to thermal cycling (TC) involving alternate immersion in a 4°C water bath and a 60°C water bath for 1 minute each, repeated 20,000 times, and the shear bond strength was measured. The tensile bond strength after this thermal cycling was used to evaluate bond durability. The results are presented in Table 1 as "After 20,000 TC cycles", Y (unit: MPa).

In this test, samples were considered acceptable when the adhesive properties after 1 day at 37°C (X) was 39 MPa or more, and X/Y (%) was 48% or more.

**[Table1]**

| | Raw materials | | | Manufacturing conditions | | | | | | Zirconia composite sintered body | | | | | | | | |
|---|---|---|---|---|---|---|---|---|---|---|---|---|---|---|---|---|---|---|
| | | | | | | | | | | Content of components | | | | | | | | Surface conditions |
| | ZrO2, stabilizer (Y203), Nb2O5 or Ta2O5 | Capping agent | Additive | Primary sintering conditions | | HIP conditions | | Tempering conditions | | Unit [mol%] | | | | | Elements or ions derived from capping agent [mol%] | Additive (TiO2 or Al2O3) [mass%] | A/B | |
| | | | | Highest sintering temp. [°C] | Hold time [hr] | Temp. [°C] | Time [hr] | Temp. [°C] | Time [hr] | (1) ZrO2 and HfO2 | (2) Y2O3 (A) | (3) Nb2O5 (B) | (4) Ta2O5 (B) | (1) to (4) in total | | | | |
| Ex. 1 | ZrO2, Y2O3, Nb2O5 | NaOH | TiO2 | 1550 | 2 | 1450 | 2 | 700 | 60 | 90.30 | 5.30 | 4.40 | 0.00 | 100.00 | 0.18 | 3.00 | 1.20 | AS surface |
| Ex. 2 | ZrO2, Y2O3, Nb2O5 | NaOH | TiO2 | 1550 | 2 | 1450 | 2 | 700 | 60 | 90.30 | 5.30 | 4.40 | 0.00 | 100.00 | 0.18 | 3.00 | 1.20 | SB surface after AS |
| Ex. 3 | ZrO2, Y2O3, Nb2O5 | NaOH | TiO2 | 1550 | 2 | 1450 | 2 | 700 | 60 | 91.50 | 5.32 | 3.18 | 0.00 | 100.00 | 0.33 | 3.00 | 1.67 | AS surface |
| Ex. 4 | ZrO2, Y2O3, Nb2O5 | None | TiO2 | 1550 | 50 | 1450 | 2 | 700 | 60 | 90.30 | 5.30 | 4.40 | 0.00 | 100.00 | 0.00 | 3.00 | 1.20 | AS surface |
| Ex. 5 | ZrO2, Y2O3, Ta2O5 | NaOH | TiO2 | 1550 | 2 | 1450 | 2 | 700 | 60 | 94.08 | 5.47 | 0.00 | 0.45 | 100.00 | 0.33 | 3.00 | 12.08 | AS surface |
| Ex. 6 | ZrO2, Y2O3 | NaOH | None | 1550 | 2 | 1450 | 2 | 700 | 60 | 94.50 | 5.50 | 0.00 | 0.00 | 100.00 | 2.00 | 0.00 | - | AS surface |
| Ex. 7 | ZrO2, Y2O3, Nb2O5 | NaOH | None | 1550 | 2 | 1450 | 2 | 700 | 60 | 90.30 | 5.30 | 4.40 | 0.00 | 100.00 | 0.18 | 0.00 | 1.20 | AS surface |
| Ex. 8 | ZrO2, Y2O3, Nb2O5 | NaOH | TiO2 | 1450 | 2 | 1450 | 2 | 700 | 60 | 90.30 | 5.30 | 4.40 | 0.00 | 100.00 | 0.18 | 3.00 | 1.20 | AS surface |
| Ex. 9 | ZrO2, Y2O3, Nb2O5 | NaOH | TiO2 | 1550 | 50 | 1450 | 2 | 700 | 60 | 90.30 | 5.30 | 4.40 | 0.00 | 100.00 | 0.18 | 3.00 | 1.20 | AS surface |
| Com. Ex. 1 | ZrO2, Y2O3 | - | - | 1550 | 2 | - | - | - | - | 94.50 | 5.50 | 0.00 | 0.00 | 100.00 | - | - | - | AS surface |
| Com. Ex. 2 | ZrO2, Y2O3 | - | - | 1550 | 2 | - | - | - | - | 94.50 | 5.50 | 0.00 | 0.00 | 100.00 | - | - | - | SB surface after AS |

| | | | | | | | | | | Properties | | | | | | | | |
|---|---|---|---|---|---|---|---|---|---|---|---|---|---|---|---|---|---|---|
| ZrO2, stabilizer (Y2O3), Nb2O5 or Ta2O5 | Capping agent | Additive | | Primary sintering conditions | | HIP conditions | | Tempering conditions | | Monoclinic fraction [%] | Average crystal grain size [µm] | Degree of penetration [mm] | Surface roughness Ra [µm] | Mean spacing of irregularities RSm [µm] | | Bond strength [MPa] | | |
| | | | Highest sintering temp. [°C] | Hold time [hr] | Temp. [°C] | Time [hr] | Temp. [°C] | Time [hr] | After 1 day at 37°C X | | | | | | | After 20,000 TC cycles Y | X/Y [%] | |
| Ex. 1 | ZrO2, Y2O3, Nb2O5 | NaOH | TiO2 | 1550 | 2 | 1450 | 2 | 700 | 60 | 23 | 2.1 | 3.5 | 4.9 | 263 | | 39.9 | 19.7 | 49.4 |
| Ex. 2 | ZrO2, Y2O3, Nb2O5 | NaOH | TiO2 | 1550 | 2 | 1450 | 2 | 700 | 60 | 23 | 2.0 | 3.5 | 5.1 | 378 | | 39.4 | 30.6 | 77.7 |
| Ex. 3 | ZrO2, Y2O3, Nb2O5 | NaOH | TiO2 | 1550 | 2 | 1450 | 2 | 700 | 60 | 35 | 1.7 | 5.0 | 5.8 | 134 | | 42.1 | 25.4 | 60.3 |
| Ex. 4 | ZrO2, Y2O3, Nb2O5 | None | TiO2 | 1550 | 50 | 1450 | 2 | 700 | 60 | 37 | 7.3 | 3.0 | 4.0 | 536 | | 40.2 | 19.6 | 48.8 |
| Ex. 5 | ZrO2, Y2O3, Ta2O5 | NaOH | TiO2 | 1550 | 2 | 1450 | 2 | 700 | 60 | 50 | 2.3 | 4.0 | 4.9 | 356 | | 40.1 | 19.6 | 48.9 |
| Ex. 6 | ZrO2, Y2O3 | NaOH | None | 1550 | 2 | 1450 | 2 | 700 | 60 | 40 | 0.8 | 2.8 | 3.3 | 361 | | 39.8 | 19.2 | 48.2 |
| Ex. 7 | ZrO2, Y2O3, Nb2O5 | NaOH | None | 1550 | 2 | 1450 | 2 | 700 | 60 | 34 | 2.3 | 3.9 | 4.9 | 289 | | 40.0 | 19.6 | 49.0 |
| Ex. 8 | ZrO2, Y2O3, Nb2O5 | NaOH | TiO2 | 1450 | 2 | 1450 | 2 | 700 | 60 | 36 | 1.3 | 4.9 | 5.6 | 138 | | 41.5 | 23.8 | 57.3 |
| Ex. 9 | ZrO2, Y2O3, Nb2O5 | NaOH | TiO2 | 1550 | 50 | 1450 | 2 | 700 | 60 | 34 | 7.2 | 2.8 | 4.1 | 512 | | 40.3 | 19.5 | 48.4 |
| Com. Ex. 1 | ZrO2, Y2O3 | - | - | 1550 | 2 | - | - | - | - | 0 | 1.5 | 0.0 | 2.5 | 934 | | 35.3 | 0.0 | 0.0 |
| Com. Ex. 2 | ZrO2, Y2O3 | - | - | 1550 | 2 | - | - | - | - | 0 | 1.6 | 0.0 | 2.8 | 880 | | 40.1 | 19.0 | 47.3 |

| | | | | | | | | | | | | | | | | | | |
|---|---|---|---|---|---|---|---|---|---|---|---|---|---|---|---|---|---|---|
| * AS: machined, SB: sandblasted | | | | | | | | | | | | | | | | | | |

These results confirmed that the machinable zirconia composite sintered bodies of the present invention can exhibit excellent adhesive properties without sandblasting after machining. In Example 2, it was confirmed that even higher bond durability can be achieved with sandblasting after machining.

In contrast, the initial adhesive properties were low and the bond durability was notably poor in Comparative Example 1 in which machining was not followed by sandblasting.

In Comparative Example 2, it was not possible to achieve adequately high bond durability and the bond durability was inferior, despite sandblasting performed after machining.

### INDUSTRIAL APPLICABILITY

A zirconia composite sintered body of the present invention can exhibit excellent adhesive properties without sandblasting after machining. This makes a zirconia composite sintered body of the present invention particularly useful as a dental prosthesis or other dental materials intended for dental treatment.

## Claims

1. A dental zirconia composite sintered body comprising monoclinic zirconia and having a monoclinic abundance fₘ of more than 10% and 60% or less as represented by the following formula (1), ${f}_{m} = {l}_{28} / \left({l}_{28}+{l}_{30}\right) \times 100$ (1), wherein fₘ represents the monoclinic abundance (%), I₂₈ represents the integrated intensity of the peak near 2θ = 28°, where the main peak of the monoclinic zirconia appears in XRD measurement, and I₃₀ represents the integrated intensity of the peak near 2θ = 30°, where the main peak of tetragonal or cubic zirconia appears in XRD measurement.

2. The dental zirconia composite sintered body according to claim 1, wherein the monoclinic zirconia is present at the surface.

3. The dental zirconia composite sintered body according to claim 1 or 2, which has a surface roughness Ra of 3.0 to 8.0 µm.

4. The dental zirconia composite sintered body according to claim 1 or 2, which has an average crystal grain size of 0.5 to 10 µm.

5. The dental zirconia composite sintered body according to claim 1 or 2, which further comprises a stabilizer capable of preventing a phase transformation of zirconia.

6. The dental zirconia composite sintered body according to claim 5, wherein the content of the stabilizer is 1 to 12 mol% in total 100 mol% of zirconia and the stabilizer.

7. The dental zirconia composite sintered body according to claim 5, wherein the stabilizer is yttria.

8. The dental zirconia composite sintered body according to claim 5, which further comprises Nb₂O₅ and/or Ta₂O₅.

9. The dental zirconia composite sintered body according to claim 8, wherein the content of the Nb₂O₅ and/or Ta₂O₅ is 1 to 9 mol% in total 100 mol% of zirconia, the stabilizer, Nb₂O₅, and Ta₂O₅.

10. The dental zirconia composite sintered body according to claim 8, which further comprises an element or ion derived from a capping agent.

11. The dental zirconia composite sintered body according to claim 10, wherein the content of the element or ion derived from a capping agent is more than 0 mol% and 5 mol% or less relative to total 100 mol% of zirconia, the stabilizer, Nb₂O₅, and Ta₂O₅.

12. The dental zirconia composite sintered body according to claim 10, wherein the element or ion derived from a capping agent belongs to periods 2 to 7 of the periodic table, and is an element or an ion thereof having a first ionization energy lower than that of a group 18 element of the same period, and/or an element or an ion thereof having a high electron affinity.

13. The dental zirconia composite sintered body according to claim 10, wherein the element or ion derived from a capping agent comprises at least one element or an ion thereof selected from the group consisting of Cu, Ag, Li, Na, K, Rb, Cs, Fr, At, I, Br, Cl, and F.

14. The dental zirconia composite sintered body according to claim 11, wherein the element or ion derived from a capping agent comprises at least one element selected from the group consisting of Li, Na, and K.

15. The dental zirconia composite sintered body according to claim 8, which further comprises TiO₂ and/or Al₂O₃.

16. A dental kit for conservative restoration, comprising the dental zirconia composite sintered body of claim 1 or 2 in combination with a dental cement.

17. The dental kit for conservative restoration according to claim 16, wherein the dental cement comprises an acidic component.

18. The dental kit for conservative restoration according to claim 17, wherein the acidic component comprises a monomer having an acidic group.

19. The dental kit for conservative restoration according to claim 18, wherein the monomer having an acidic group comprises at least one selected from the group consisting of a phosphoric acid group, a pyrophosphoric acid group, a thiophosphoric acid group, a phosphonic acid group, a carboxylic acid group, and a sulfonic acid group.

20. A method for bonding the dental zirconia composite sintered body of claim 1 or 2, comprising bonding using a dental cement, without sandblasting.
